# EUROPEAN PATENT SPECIFICATION

(11) **EP 4 487 805 B1**
(45) Date of publication and mention of the grant of the patent: **03.06.2026**
(21) Application number: 24186702.7
(22) Date of filing: 05.07.2024
(51) Int. Cl.: A61B 34/20, A61B 5/06, A61B 90/98, A61B 18/14, A61B 17/00, A61M 25/00

(54) **GUIDING SHEATH SYSTEM WITH POSITION SENSING**
FÜHRUNGSHÜLLENSYSTEM MIT POSITIONSERFASSUNG
SYSTÈME DE GAINE DE GUIDAGE AVEC DÉTECTION DE POSITION

(30) Priority: 07.07.2023 US 202363525494 P; 28.12.2023 US 202318399230
(43) Date of publication of application: 08.01.2025
(73) Proprietor: Biosense Webster (Israel) Ltd., 2066717 Yokneam (IL)
(72) Inventor: STANLEY, Mark T., Irvine, 92618 (US); TANG, Raymond Yue-Sing, Irvine, 92618 (US); AGNEW V, William James, Irvine, 92618 (US); KIM, Amie, Irvine, 92618 (US); SALAHUDDIN, Afrah, Irvine, 92618 (US); ABOYTES, Donald, Irvine, 92618 (US); KOZOKARO, Kfir, Irvine, 92618 (US); BANANDO, Michael D., Irvine, 92618 (US); SIU, Zachary, Irvine, 92618 (US)
(74) Representative: Carpmaels & Ransford LLP

(56) References cited:
- US-A1- 2005 261 568
- US-A1- 2007 135 803
- US-A1- 2022 096 149
- US-A1- 2022 202 500
- US-A1- 2023 015 694

## Description

### CROSS REFERENCE TO RELATED APPLICATION(S)

This application claims priority to and the benefit of U.S. Provisional Application No. 63/525,494, filed July 7, 2023.

### FIELD OF INVENTION

This invention relates to guiding sheath and intralumenal devices used with guiding sheaths, such as dilators, therapeutic and diagnostic catheters and transseptal needles.

### BACKGROUND

Cardiac arrhythmia is irregular beating of the heart caused by aberrant electrical signals. Arrhythmias can reduce quality of life and carry increased risk of stroke and heart failure. Arrythmias can be located and identified via diagnostic catheters. These catheters can be used to create electroanatomical maps to help electrophysiologists understand the pathology and plan and deliver therapy which can include ablation via therapeutic catheters.

Electrophysiology (EP) catheters, whether diagnostic or therapeutic, are guided by guiding sheaths which are well known for use in facilitating pathway within a patient's vasculature, typically through a femoral artery and aorta to ultimately gain access to the four chambers of the heart. For example, CARTO .VIZIGO^{®} Bi-Directional Guiding Sheath by Biosense Webster, Inc., Irvine, California, allows for sheath visualization in real-time, three dimensional maps via an imaging electromagnetic system, such as The CARTO^{®} 3 System by Biosense Webster, Inc. Irvine, California. The System enables electrophysiologists to build accurate 3-D electroanatomical maps of the heart and is designed to assist electrophysiologists navigate EP vascular instruments, such as sheaths, catheters, dilators and other probe devices, inside the heart by pinpointing the exact position (location and orientation) of the distal ends of these EP vascular instruments during diagnostic and therapeutic procedures.

For visualization of an EP vascular instrument with The CARTO^{®} 3 System, the instrument carries in or near its distal end an electromagnetic position sensor with three coils, each responsive to a respective magnetic field generators positioned under the patient's bed. Each coil generates a signal that is transmitted by a respective lead wire extending from the distal end of the instrument along its entire length to and through a control handle of the instrument and into an electrical connector which connects with the System for processing and generation of a depiction of the instrument on a monitor displaying a 3-D anatomical map of the heart. Fluoroscopy is often use in lieu of or in addition to electromagnetic position sensing. The ionizing radiation poses a risk to patients and to electrophysiologists who must wear heavy lead-filled garments. Moreover, the view provided is limited to 2D.

The shaft and distal tip of EP vascular instruments are small by necessity and thus their construction and assembly require highly skilled workers. Moreover, as the field of cardiac electrophysiology advances, more and more components are housed or carried on the instruments' distal ends where space is already at a premium. Furthermore, because the distal ends are advanced into the heart, medical safety requirements are stringent so as to minimize the risk of inadvertent detachment of components from the distal tip or other avoidable traumatic injury to the heart tissue. And, where electrical components in the distal end are connected to lead wires, breakage in the lead wires results in instrument failure.

For intralumental devices, such as dilators and transseptal needles that are advanced into patient vasculature, visualization is often not available. Thus, the position of the dilator and the transseptal needle is based primarily on guesswork and best estimates of the operators handling these devices. While identification of these devices is possible with fluoroscopy with confirmation by ultrasound, reduction of overall fluoroscopy time is in the interest of the patient's health.

Accordingly, applicants recognized that there is a need to provide EP vascular instruments, especially those used with guiding sheaths, that allow position sensing and visualization with less demands on construction, assembly efforts and use of space in the distal end while also minimizing the need for lead wires that extend along the length of the instruments, with the understanding that improper approach of intralumenal devices in the heart can result in tissue damage and ailments, such as cardiac perforation and cardiac tamponade.

US 2005/261568 A1 describes a multisensor probe for continuous real-time tissue identification. The multisensor probe includes a tissue penetrating needle, a plurality of sensors useful in characterizing tissue and a position sensor to measure the depth of the needle into the tissue being diagnosed. The sensors include but are not limited to an optical scattering and absorption spectroscopy sensor, an optical coherence domain reflectometry sensor, an electrical impedance sensor, a temperature sensor, a pO2 sensor, a chemical sensor and other sensors useful in identifying tissue. The sensors may take the form of a plurality of optical fibers extending through said needle. A retractable sheath may be disposed around the distal section of the needle to protect the needle when not in use. The sheath retracts when the probe is inserted into tissue and the position of the sheath relative to the needle may be measured to determine the needle's depth. Systems and methods for tissue identification are also provided.

US 2022/202500 A1 describes a robot-assisted endoscope system and control methods thereof, to allow a user to perform intraluminal interventional procedures using a steerable sheath. A processor generates a ghost image based on a non-real-time insertion trajectory of the sheath, and a real-time image based on a real-time insertion trajectory for inserting an interventional tool through the sheath towards the target site. A display screen outputs navigation guidance data for informing a user how to manipulate the distal section of the sheath towards the target site such that the real-time image overlaps or coincides with at least part the ghost image and the real-time insertion trajectory becomes aligned with the non-real-time insertion trajectory.

US 2007/135803 A1 describes an apparatus for use in a transluminal procedure. The apparatus, comprising, for example, a housing having a guide lumen and a seal proximal to a distal end of the housing that extends across and completely seals the guide lumen; a fixation element in the housing and adapted to secure the distal end of the housing to tissue; and a channel extending through the side wall of the housing having an outlet in communication with the lumen distal of the seal. Methods are also provided. For example, a method includes, performing a transluminal procedure by: securing a datum and position indicator to a wall of a target lumen; forming an opening in the wall; advancing an instrument through the opening; and tracking the advancement of the instrument using the datum and position indicator.

### SUMMARY OF THE DISCLOSURE

The invention is defined in the independent claim. Embodiments are defined in the dependent claims.

### BRIEF DESCRIPTION OF THE DRAWINGS

These and other features and advantages of the present invention will be better understood by reference to the following detailed description when considered in conjunction with the accompanying drawings. It is understood that selected structures and features have not been shown in certain drawings so as to provide better viewing of the remaining structures and features.
FIG. 1A is top view of a guiding sheath system with position sensing and at least one intralumenal device extending therethrough, according to an embodiment.
FIG. 1B is a top view of the guiding sheath system of FIG. 1A with a distally-advanced first intralumenal device with an exposed distal end.
FIG. 1C is a top view of the guiding sheath system of FIG. 1B with distally-advanced first and second intralumenal devices, both with exposed distal ends.
FIG. 1D is a side cross-sectional view of the distal ends of FIG. 1C in deflection.
FIG. 1E is a cross-sectional view of the distal end of the first intralumenal device at an approach distance AD1 (in solid lines) and at a deployed distance DD1 (in broken lines) relative to a guiding sheath, according to an embodiment.
FIG. 1F is a cross-sectional view of the distal end of the second intralumenal device at an approach distance AD2 (in solid lines) and at a deployed distance DD2 (in broken lines) relative to the first intralumenal device, according to an embodiment.
FIG. 1G is a side cross-sectional view of a proximal portion of the second intralumenal device coextensive with a proximal portion of the first intralumenal device that is extending through the guiding sheath, according to an embodiment.
FIG. 2A is a perspective view of the guiding sheath control handle, partially broken away to show a pathway aligned with sensors, the pathway configured to receive first and second intralumenal devices carrying emitters, according to an embodiment.
FIG. 2B a perspective view of the first and second intralumenal devices of FIG. 2A, in coextension.
FIG. 3 is a schematic view of relative configurations and geometries of the guiding sheath and first and second intralumenal devices, according to an embodiment.
FIG. 4A is an illustration of a guiding sheath system with advanced EP imaging, according to one embodiment.
FIG. 4B is a schematic block diagram illustrating operation of the system of FIG. 4A, according to one embodiment.
FIG. 4C is a schematic block diagram illustrating operation of the system of FIG. 4A, according to another embodiment.
FIGS. 5A, 5B and 5C are end-sectional views of first and second intralumenal devices with respective emitters, coextensive through a guiding sheath with sensors, and corresponding voltages generated by the sensors in response to the emitters in different rotational angles about the longitudinal axes of the intralumenal devices, according to an embodiment.
FIGS. 6A and 6B are side cross-sectional views of an intralumenal device with magnetic emitters in a relative configuration with a guiding sheath with sensors, and corresponding voltages generated by the sensors in response to different longitudinal positions and orientations of the emitters, according to an embodiment.
FIGS. 7A and 7B are end-sectional views of an intralumenal device with a ring emitter extending through a guiding sheath with sensors, the ring emitter in different rotational angles relative to sensors.
FIG. 8A is a perspective view of the guiding sheath control handle, partially broken away to show a pathway aligned with optical sensors configured to receive an intralumenal device carrying optical emitters, according to an embodiment.
FIG. 8B is a perspective view of the intralumenal device of FIG. 8A.
FIG. 9 is a graph of photodiode voltage versus optically-readable pattern units.
FIG. 10 is a flow chart illustrating steps of position sensing an intralumenal device at its proximal portion to determine the position of its distal end relative to a guiding sheath through which the intralumenal device extends, according to an embodiment.
FIG. 11 is a flow chart illustrating steps of position sensing an intralumenal device at its proximal portion to determine the position of its distal end relative to a guiding sheath through which the intralumenal device extends, according to another embodiment.
FIG. 12 is a flow chart illustrating steps of position sensing one or more intralumenal devices at their respective proximal portions to determine the position of their respective distal ends relative to a guiding sheath through which the one or more intralumenal devices extend, according to yet another embodiment.
FIG. 13 is an illustration of an EP image as displayed on a display monitor depicting first and second intralumenal devices with distal ends deployed past the distal end of the guiding sheath within a chamber of a patient's heart.
FIG. 14A, FIG. 14B and FIG. 14C are side cross-sectional views of a guiding sheath control handle and an intralumenal device in distal advancement therethrough, each with electrically conductive members configured to come into contact to form a completed electrical circuit.
FIG. 15A is a schematic diagram of the completed electrical circuit of FIG. 14C. formed by first, second and third conductive members in contact.
FIG. 15B is is a graph of linear displacement versus electrical conductivity of the electrical circuit of FIG. 14, FIG. 14B and FIG. 14C.
FIG. 16A, FIG. 16B and FIG. 16C are side cross-sectional views of a guiding sheath control handle and an intralumenal in distal advancement therethough, each with arrays of electrically conductive members configured to come into contact to form respective completed electrical circuits.
FIG. 17 is a schematic diagram of the respective electrical circuits of the arrays of FIG. 16A, each with a circuit sensor.
FIG. 18 is a graph of linear displacement versus impedance.
FIG. 19 is a schematic diagram of a circuit for measuring impedance of a completed electrical circuit formed by first, second and third conductive members in contact
FIG. 20A, FIG. 20C, FIG. 20E, FIG. 20G and FIG. 20I are side cross-sectional views of an intralumenal device in distal advancement through a lumened device in penetrating a septum.
FIG. 20B, FIG. 20D, FIG. 20F, FIG. 20H and FIG. 20J are visualization of the intralumenal and lumened devices of FIG. 20A, FIG. 20C, FIG. 20E, FIG. 20G and FIG. 20I, with a graphical element representing an exposed distal portion of the intralumenal device distal of a distal end of the lumened device.

### DETAILED DESCRIPTION OF THE PREFERRED EMBODIMENTS

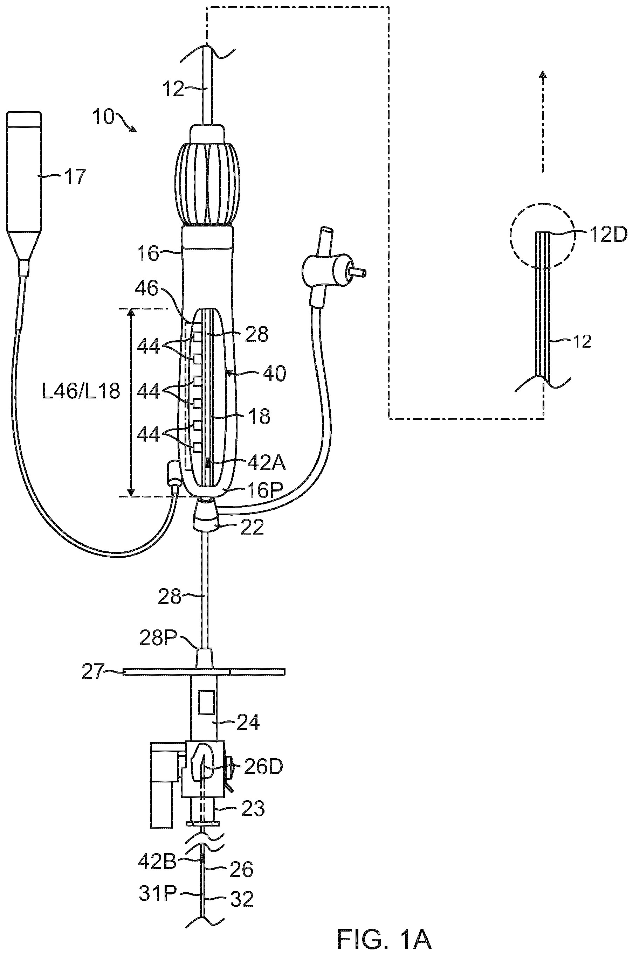

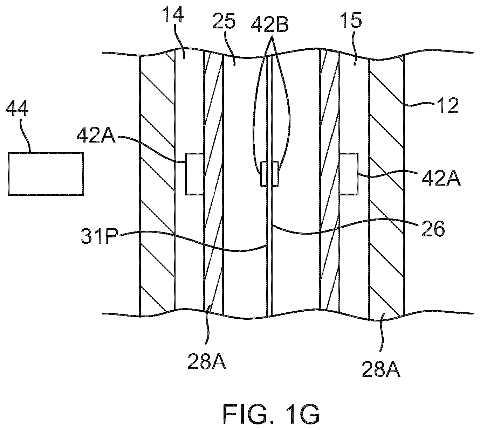

Referring to **FIG. 1A****,** **FIG. 2A****,** and **FIG. 3****,** in some embodiments of the present invention, a guiding sheath 10 includes an elongated flexible catheter tube or sheath 12, and a control handle 16 proximal of sheath 12. The sheath 12 is configured with a lumen 14 through which various intralumenal devices can traverse and be guided through a patient's vascular to ultimately reach chambers in the patient's heart. The control handle 16 at its proximal end has an electrical connector 17 for connection to a remote control and processing system in transmitting electrical signals for energizing components of the devices and the guiding sheath and/or receiving signals generated or detected by components of the intralumenal devices and the guiding sheath.

The lumen 14 of the sheath 12 is in communication with a longitudinal pathway 18 provided in the control handle 16. The pathway 18 has a length L18 and is configured to receive an intralumenal device that can traverse from the pathway 18 of the control handle 16 into the lumen 14 of the sheath 12. In some embodiments, a proximal end of the pathway 18 is defined by a hemostatic valve 22 extending proximally from a proximal end of the control handle 16, where access into the proximal end of the pathway 18 is through a fluid-tight seal of the valve 22 that permits entry and passage of the intralumenal device without fluid leaking out or air entering the valve 22. If lumened, a first (or outer) intralumenal device 24 that is advanced through the pathway 18 of the control handle 16 and further through the lumen 14 of the sheath 12 allows a second (or inner) intralumenal device 26 to be passed through a lumen 25 of the first intralumenal device 24. It is understood that either of the intralumenal devices may include an end effector, for example, a septum needle, an electrode assembly configured to map an organ, and/or an ablation electrode assembly.

In some embodiments, the first intralumenal device 24 is a lumened catheter or dilator that is advanced through the guiding sheath 10 into a patient's heart and followed by a transseptal needle or wire as the second intralumenal device 26 that is advanced through a lumen 25 of the catheter or dilator 24 into the patient's heart.

The guiding sheath 10 including the sheath 12 and the control handle 16 has a total length LT from a proximal end 16P of the control handle to a distal end of the sheath 12D. In some embodiments, for example, where the first intralumenal device 24 is a dilator, the device has a handle 27, a valve 23 and a shaft 28. Whereas the shaft 28 is configured for insertion into the guiding sheath 10, the handle 27 and the valve 23 are configured to remain proximal of the control handle 16 of the guiding sheath 10. The shaft 28 of the device 24 has a predetermined length LIN1 between a proximal end 28P and a distal end 28D, where the length LIN1 is greater than the length LT of the guiding sheath 10 by at least a length of DD1, such that the distal end 28D of the first intralumenal device 24 has an exposed length of at DD1 past the distal end 12D of the guiding sheath 10 when the first intralumenal device 24 is sufficiently advanced within the guiding sheath for deployment at its distal end 28D (for example, with its handle 27 abutting the proximal end 16P of the control handle 16). The first intralumenal device 24 has a total length L24 between the valve 23 and the distal end 28D.

The second intralumenal device 26 has an elongated body 31 of length LIN2 between a proximal end 26P and a distal end 26D, where the length LIN2 is greater than the total length L24 of the first intralumenal device 24 such that the body 31 has at least a distal exposed length DD2 distal of the distal end 28D of the first intralumenal device 24 (if not also a proximal excess length LEX proximal of the valve 23 of the first intralumenal device 24) when the second intralumenal device 26 is extending through the first intralumenal device 24 with its distal end 26 deployed at the treatment site within the patient's heart, for example, the septum. As a safety precaution, the distal exposed length DD2 of the second intralumenal device 26 may be limited by a stop 26S positioned to set a calibrated maximum advance position of the second intralumenal device 26 relative to the first intralumenal device 24 and/or the guiding sheath 10, in minimizing the risks of inadvertent tissue punctures by the distal end 26D.

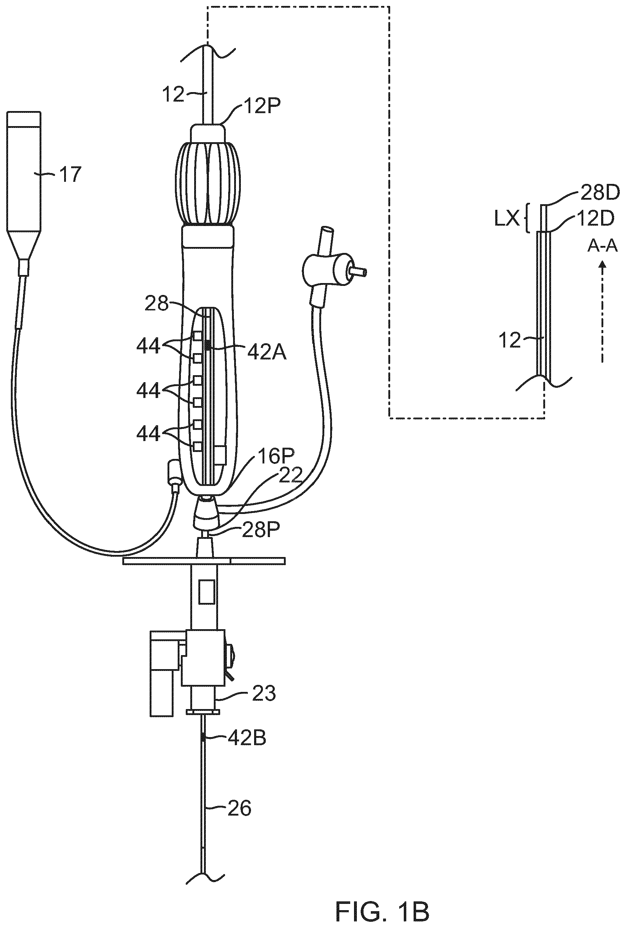

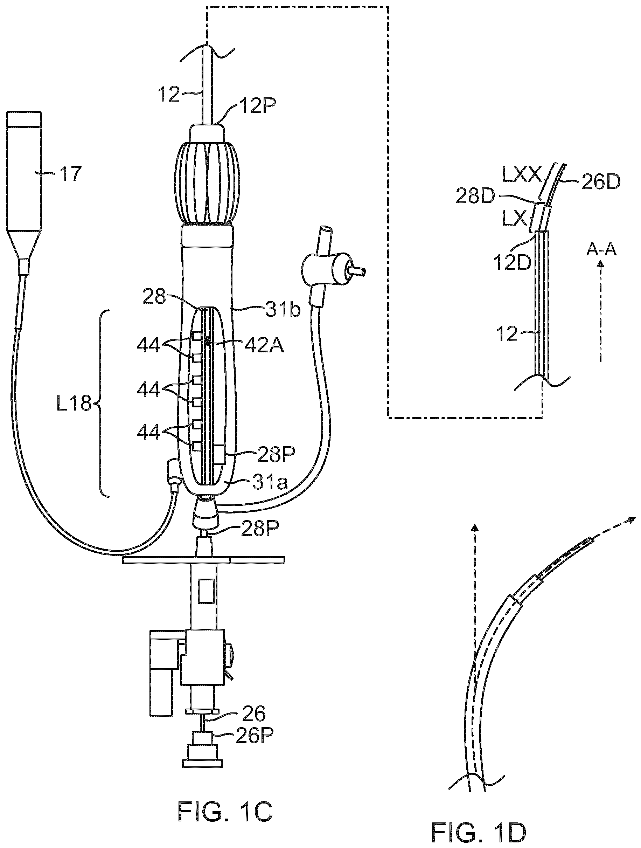

In typical use of these devices, with reference to **FIG. 1A****,** **FIG. 1B** and **FIG. 1C****,** an electrophysiologist inserts the sheath 12 of the guiding sheath 10 into the patient's femoral artery. The shaft 28 of the first intralumenal device 24 is inserted through the hemostatis valve 22 into the pathway 18 of the control handle 16 and then further advanced distally into the lumen 14 of the guiding sheath 10. The first intralumenal device 24 is advanced distally relative to the guiding sheath 10 until the distal end 28D passes the distal end 12D of the guiding sheath 10. With distal end 28D exposed, the first intralumenal device 24 is deployed and ready for diagnostic use, therapeutic use and/or receiving the second intralumenal device 26. In the latter instance, the second instrument 26 is advanced distally through the lumen 25 of the first intralumenal device 24 until the distal end 26D passes the distal end 28D of the first intralumenal device 24. With the distal end exposed 26D, the second intralumenal device 26 is deployed and ready for diagnostic and/or therapeutic use.

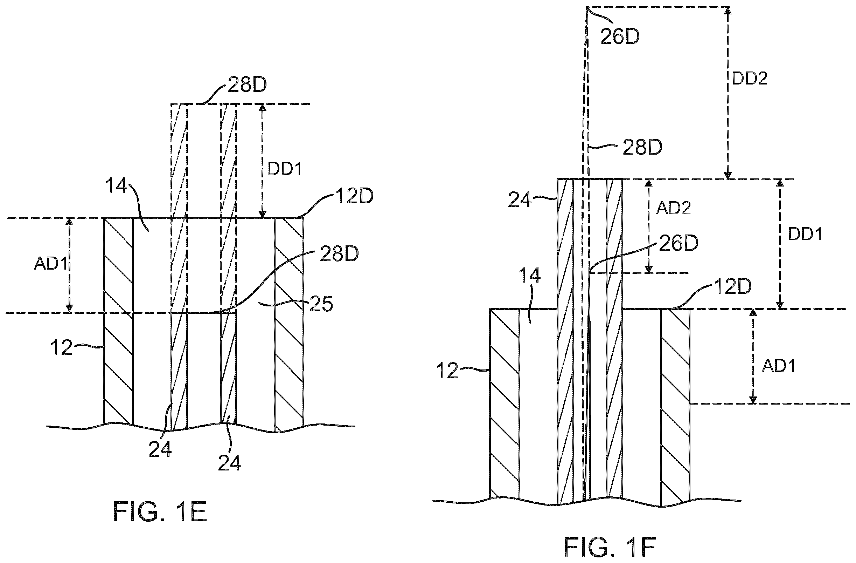

During these approach and deployment stages, the user's interest in knowing the position of the first intralumenal device 24 generally heightens when the distal end 28D is approaching the distal end 12D of the guiding sheath 10 **(****FIG. 1E****)** and advancing past it **(****FIG. 1F**). The heightened interest may continue at least as long as the distal end 28D is exposed outside of the sheath 12. Calibrated maximum distal advancement of the first intralumenal device 24 relative to the sheath 12 may be limited by the device handle 27 abutting the hemostatis valve 22 or the proximal end 16P of the control handle 16 of the guiding sheath 10 **(****FIG. 1B****).** Likewise, where the second intralumenal device 26 has been inserted into the first intralumenal device 24, the user's interest in knowing the position of the second intralumenal device 26 generally heightens when the distal end 26D is approaching the distal end 28D of the shaft 28 of the first intralumenal device 24 (in solid lines in **FIG. 1F****)** and advancing past it (in broken lines in **FIG. 1F****).** The heightened interest may continue at least as long as the distal end 26D is exposed outside of the distal end 28D of the first intralumenal device 24. Depending on the configuration of the proximal end of the second intralumenal device 26, there may be a calibrated maximum distal advancement of the second intralumenal device relative to the first intralumenal device 24 and/or the guiding sheath 10. For example, where the first intralumenal device 24 is a dilator that is advanced through the guiding sheath 10 and followed by a transseptal needle as the second intralumenal device 26 that is advanced through the dilator, both the distal ends of the dilator and the transseptal needle are narrower and/or more steeply tapered relative to the guiding sheath which can be traumatic and injurious to heart tissue if they are advanced too far distally past the distal end 12D of the sheath 12. Therefore, it is in the user's interest to track or monitor linear advancement and placement of the distal ends 28D and 26D relative to distal end 12D of guiding sheath 10 so as to minimize the risk of injury to the patient, including the risk of any inadvertent puncturing of heart tissue.

For one or more of the intralumenal devices, in particular as to their distal ends, to be visualized on a display monitor for the user, a guiding sheath system in some embodiments, includes components of an advanced electrophysiology ("EP") imaging system S that support components of a position sensing assembly 40 advantageously provided on respective proximal portions of the guiding sheath 10 and of each of the intralumenal devices 24, 26. The position sensing assembly 40 provides signals that are generated by the proximal portions of the intralumenal devices 24, 26 and transmitted to the system S via the electrical connector 17 for processing by the system in determining positions (including location and orientation) of the distal ends of the intralumenal devices 24, 26 relative to the guiding sheath 10.

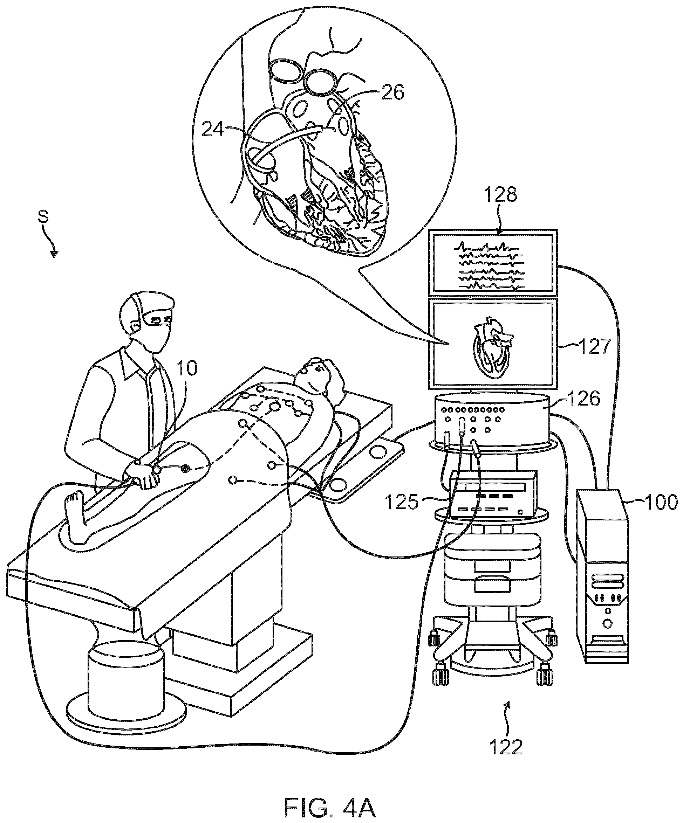

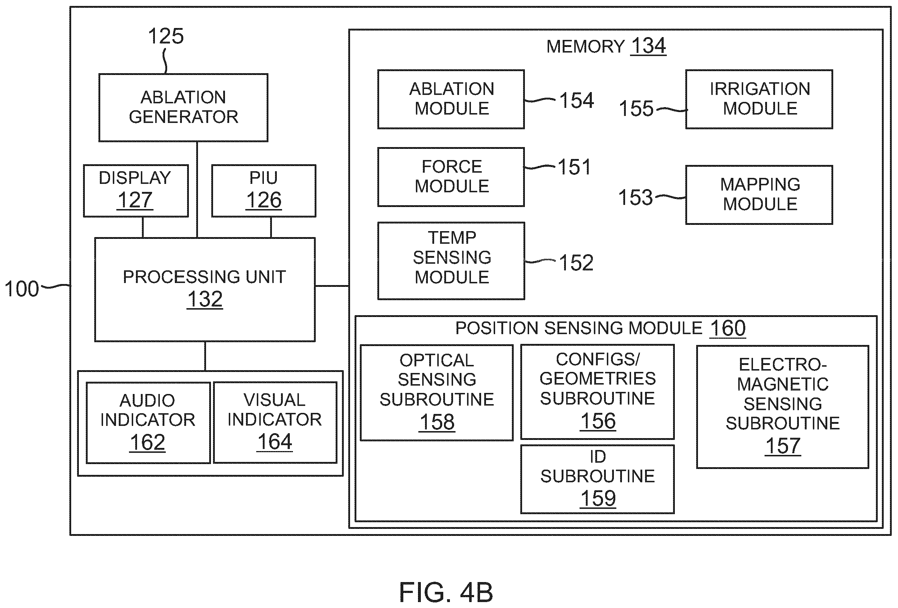

In some embodiments, with reference to **FIG. 4A** and **FIG. 4B****,** the EP imaging system S includes a console 122 which may include an ablation power generator 125, a patient interface unit (PIU) 126, and one or more displays 127 and 128 to display 3-D maps and electrograms. The operations, functions and acts of the EP imaging system S are managed by the system controller 100 that includes a processing unit 132 communicating with a memory 134 wherein is stored software for operation of the system. In some embodiments, the system controller 100 is an industry-standard personal computer including a general-purpose computer processing unit. However, in some embodiment, at least some of the operations, functions or acts of the system controller are performed using custom-designed hardware and software, such as an application specific integrated circuit (ASIC) or a field programmable gate array (FPGA). In some embodiments, the system controller is managed by an operator using a pointing device and a graphical user interface (GUI), which enable the operator to set parameters of the system. The GUI typically also displays results of the procedure to the operator on a display. The software in memory may be downloaded to the controller in electronic form, over a network, for example. Alternatively or additionally, the software may be provided on non-transitory tangible media, such as optical, magnetic or electronic storage media.

In some embodiments, the memory 134 includes multiple modules used by the processing unit 132, such as, for example, a force sensing module 151, temperature sensing module 152, a 3-D mapping module 153, an ablation module 154, and an irrigation module 155, the functions of which are known in the art, for example, for respectively, measuring tissue temperature, determining position of components within the patient's body with the use of electromagnetic navigation/position sensors responsive to external magnetic field generators positioned outside of the patient's body (for example, below the patient's bed), energizing electrodes to ablate tissue, and controlling flow of irrigation fluid to the treatment site. Further included in the memory 134 is a position sensing module 160 supporting the position sensing assembly and position sensing methods used by the processing unit 132.

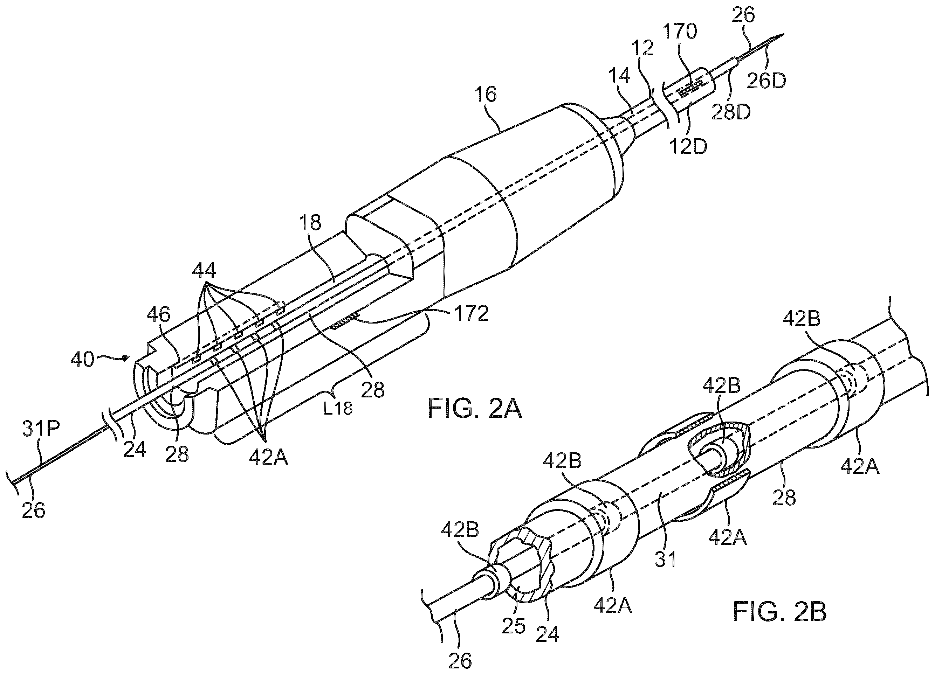

In some embodiments, with reference to **FIG. 1A****,** **FIG 2A** and **FIG 2B****,** components of the position sensing assembly 40 include at least emitter 42A advantageously situated on a proximal portion of the first intralumenal device 24 and at least one sensor 44 fixed or otherwise situated along the pathway 18 in the control handle 16 of the guiding sheath 10, such that the emitter 42A and the sensor 44 can interact with each other as the first intralumenal device moves through the pathway 18. For example, as the emitter 42A carried on the first intralumenal device 24 moves into sensing proximity of the sensor 44, signals are generated by the sensor in response to the presence and/or movement of the emitter 42A relative thereto wherein such generated signals are indicative of at least the position (linear and/or rotational) of the proximal portion of the first intralumenal device 24 relative to the control handle 16, if not also representative of the position (linear and/or rotational) of the distal end 28D of the first intralumenal device 24 due to its predetermined configurations and geometries.

In some embodiments, with reference to **FIG. 1A** **and** **FIG 2A** and **FIG 2B****,** components of the position sensing assembly 40 also include at least one emitter 42B advantageously situated on a proximal portion of the second intralumenal device 26, such that the emitter 42B and the sensor 44 can interact with each other as the second intralumenal device moves through the lumen 25 of the first intralumenal device 24. For example, as the emitter 42B carried on the second intralumenal device 26 moves into sensing proximity of the sensor 44, signals are generated by the sensor in response to the presence and/or movement of the emitter 42B relative thereto wherein such generated signals are indicative of at least the position (linear and/or rotational) of the proximal portion of the second intralumenal device 26 relative to the control handle, if not also representative of the position (linear and/or rotational) of the distal end 26D of the second intralumenal device 26 due to predetermined configurations and geometries of the intralumenal device 26 and the guiding sheath 10 .

It is understood that each of the first and second intralumenal devices 24 and 26 has respective predetermined configurations and geometries such that the spatial relationship between its respective distal end and proximal portion is known and remains generally predictable, whether or not the devices are configured for deflection unidirectionally or bidirectionally (see **FIG. 1D****).** The configurations and geometries of the first and second intralumenal devices and the guiding sheath for use with the system are stored in configurations and geometries subroutine 156 of memory 134 **(****FIG. 4B****).** Accordingly, the system can utilize the signals generated by the sensor in response to the emitter in the proximal portion of each of the first and second intralumenal devices to reliably determine the position (location and orientation) of the respective distal ends of these devices relative to the guiding sheath.

In some embodiments, with reference to **FIG. 1A** and **FIG. 2A****,** where a plurality of sensors 44 are situated in the pathway 18 of the control handle 16 of the guiding sheath, the sensors 44 are arranged linearly and spaced apart evenly to form an N-by-1 sensor array 46 that has a length L46 that extends along the pathway 18, parallel with a longitudinal axis 48 of the control handle 16. The length L46 may be generally equal to the length L18 of the pathway and thus L18 and L46 may be used interchangeably herein. N may range between 1 and 10, and preferably be 6 in some embodiments.

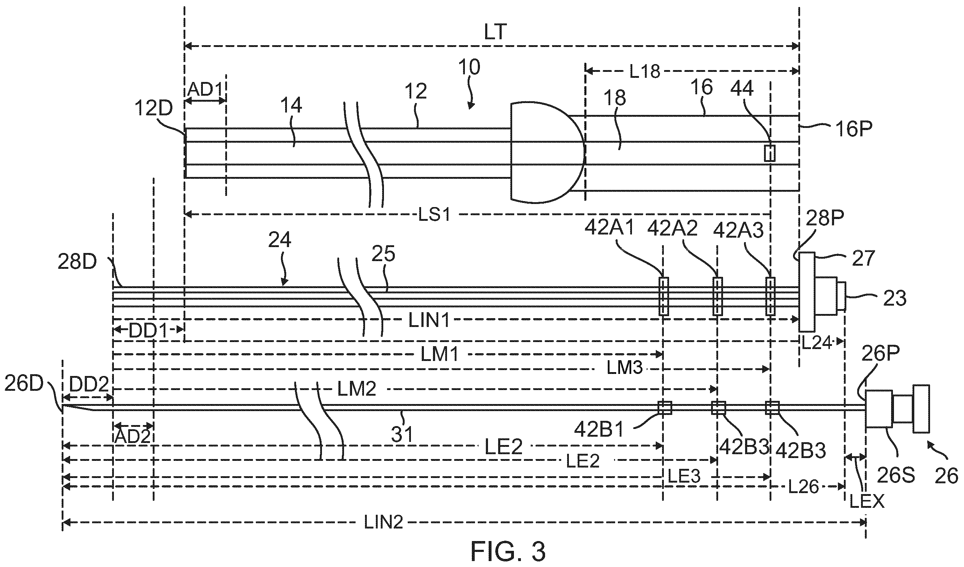

As shown in **FIG. 3****,** the one or more emitters 42A of the first intralumenal device 24 are supported on a proximal portion of the shaft 28 that is coextensive with the pathway 18 in the immediate proximity of the sensors 44 of the array 46 when the first intralumenal device 24 has been sufficiently advanced into the guiding sheath 10 for deployment past the distal end 12D of the guiding sheath 10. The one or more emitters 42B of the second intralumenal device 26 are supported on a proximal portion of an elongated body 31 of the device 26 that extends through the lumen 25 of the shaft 28 and is coextensive with the pathway 18 when the second intralumenal device 26 has been sufficiently advanced into the first intralumenal device 24 for deployment past the distal end 28D of the first intralumenal device 24.

An approach distance AD of an intralumenal device is defined herein as a predetermined distance within which a user's interest is heightened when a distal tip of the intralumenal device is distally approaching the distal end of the guiding sheath (or of a first or outer intralumenal device). An exposed (or deployed) length DD of an intralumenal device is defined herein as a predetermined maximum distance that a user permits a distal end of the intralumenal device to extend past the distal end of the guiding sheath (or of a first or outer intralumenal device),

In embodiments where the length L46 of the sensor array 46 is less than either of (i) exposed DD1 of the first intralumenal device 24 or (ii) exposed length DD2 of the second intralumenal device 26, the respective intralumenal device 24, 26 may be configured to carry multiple emitters spread along the respective proximal portion for a distance equal to or greater than the length L45 to compensate for the shortage in length of the sensor array L46 relative to the greater exposed length DD1 and/or DD2, with the separation distance between each emitter being dependent at least in part on the length L46 of the sensor array.

In some embodiments, where the length L46 of the sensor array 46 in the control handle 16 of the guiding sheath 10 is greater than each of (i) approach distances AD1 of the first intralumenal device 24 and AD2 of the second intralumenal device 26, as shown in **FIG. 3****,** and (ii) deployed distances DD1 of the first intralumenal device 24 and DD2 of the second intralumenal device 26, as shown in **FIG. 3****,** each intralumenal device may carry a single emitter, as scaling is not needed. However, it is understood that an intralumenal device is not limited to carrying a single emitter and that each intralumenal device may carry multiple emitters, as appropriate or desired.

For finer detection and greater resolution in position sensing by the system, it is understood that the N plurality of sensors 44 in the array 46 and/or the separation distance between adjacent sensors 44 can be varied as desired or appropriate. In some embodiments, the separation distance between adjacent sensor members 44 in the array 46 is equal or less than the lesser of approach distances AD1, AD2. In some embodiments, the separation distance is equal or less than the lesser of deployed distances DD1, DD2. In some embodiments, the separation distance is equal or less than the lesser of the approach distances AD1, AD2 and the deployed distances DD1, DD2.

In some embodiments, the separation distance between adjacent sensors 44 is less than the distance or field of detection of a sensor 44 so that the detection fields of two adjacent sensors include an overlap and thus an emitter is detected by at least one sensor at any given time while the emitter is within the detection of the sensor array 46.

As described herein, detection and determination of linear movement and position of an outer intralumenal device and an inner intralumenal device relative to the control handle of the guiding sheath are enabled by interaction between the emitters 42A, 42B and the sensor array 46 as the intralumenal devices move along the pathway of the control handle. In some embodiments, the emitters may include magnetic field generating members, for example, permanent or temporary magnets, and the sensors include magnetic field sensors, for example, Hall sensors or coil-based sensors.

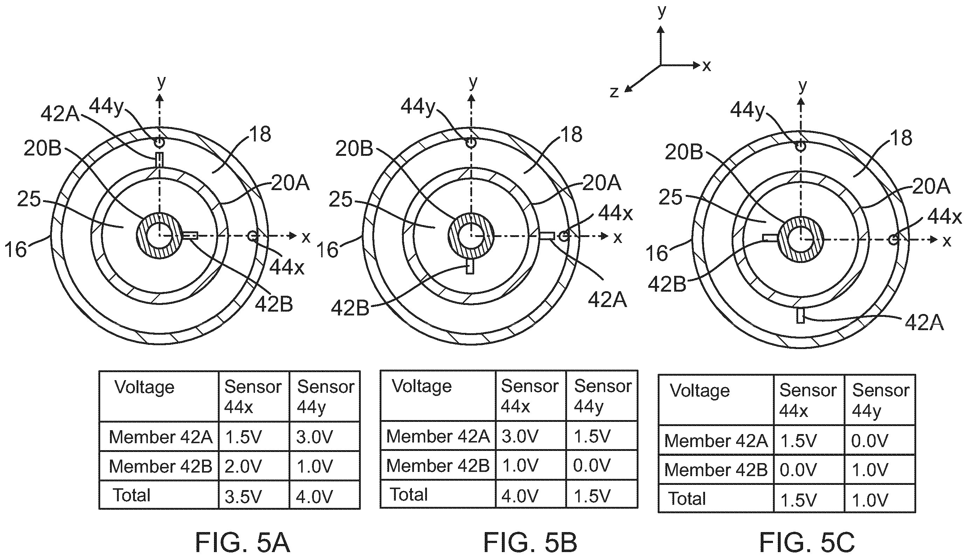

In embodiments where the emitters 42A, 42B are magnetic rings that are carried circumferentially on outer surfaces of the intralumenal devices and are magnetically radially symmetrical about the longitudinal axis of the intralumenal devices, the rings may not adequately provide information on the rotational position (movement or displacement, used interchangeably herein) of the intralumenal devices to the sensor array along the pathway. Thus, in some embodiments, as shown in **FIG. 5A, FIG. 5B and FIG. 5C****,** for detection and determination of rotational movement and position of the intralumenal devices 24, 26 about their longitudinal axis Z, additional magnetic field generating members 42A and 44B are situated off the Z axis on the intralumenal devices 24, 26, respectively, configured in the form of off-axis tabs for interaction with orthogonal (or off-angle at greater or less than 90 degrees) sensors 44x and 44y for detecting and determining "roll" movement and position. The sensors 44x and 44y are located in the same axial position (same x/y plane) along the longitudinal axis Z of the control handle 16 but at different angular positions about the longitudinal axis Z. As each of the intralumenal devices 24 and 26 rotates or otherwise assumes different radial angles about the Z axis relative to the sensor 44x (along the x axis) and sensor 44y (along the y axis), different and distinguishable signals are generated in response to the different angular positions of each member 42A, 42B relative to each of the orthogonal sensors. Thus, the orthogonal (or off angle) sensors 44x, 44y can provide signals representative of rotational movement and displacement in the x/y plane of each intralumenal device. In embodiments where pairs or sets of sensors 44x and 44y are located at different axial locations along the z axis, multiple sensors 44x extending in the Z axis direction can form a sensor array 46x and multiple sensors 44y extending in the Z axis direction can form a sensor array 46y, where these arrays can generate signals representative of both linear and rotational movement and position of each intralumenal devices as they advance through the guiding sheath 10.

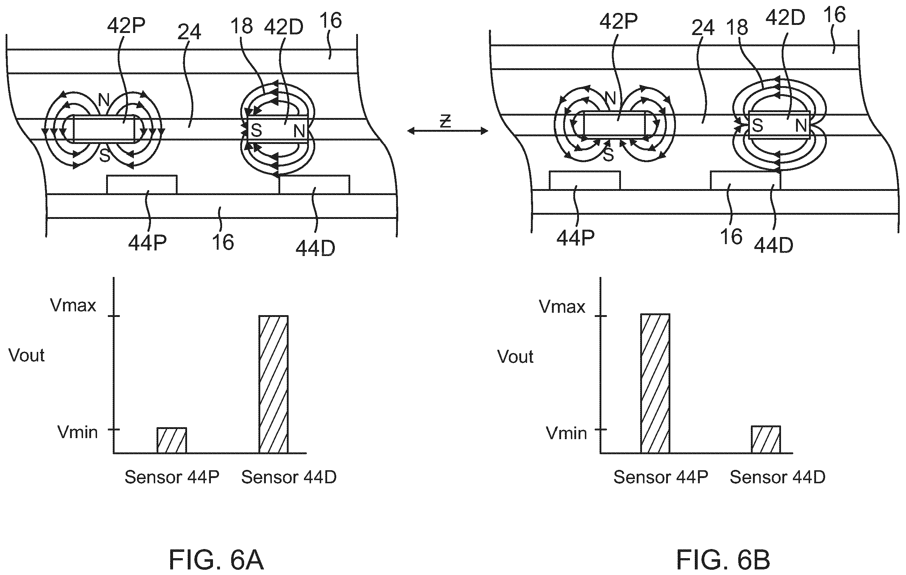

In some embodiments, with reference to **FIG. 6A** and **FIG. 6B****,** a pathway 18 of a control handle 16 has a single linear array 46 with a proximal sensor 44P and a distal sensor 44D. Extending therethrough is an intralumenal device 24 with a proximal magnetic field emitter 42P and a distal magnetic field emitter 42D configured so that their respective magnetic dipoles or fields are orthogonal to each other. For example, as depicted, the proximal emitter 42P generates magnetic field lines that are perpendicular to the longitudinal axis Z of the intralumenal device 24, whereas the distal emitter 42D generates magnetic field lines that are parallel to the longitudinal axis Z. The emitters 42P, 42D may be embedded in the shaft of the intralumenal device. In some embodiments, the distal emitter 42P is configured with its magnetic field lines parallel to the longitudinal axis Z which produce a changing voltage in a sensor from 0.0V to 3.0V when moved distally past the sensor and a changing voltage from 3.0V to 0.0V when moved proximally past the sensor. As such, the signals generated by the distal sensor 44D can be representative of linear position or movement of the intralumenal device 20, and direction of movement. In some embodiments, the proximal emitter 42D is configured with its magnetic field lines orthogonal to the longitudinal axis Z which produce a changing voltage from 0.0V to 3.0V when the intralumenal device 24 is rotated 180 degrees about its longitudinal axis relative to a sensor. As such, the signals generated by the proximal sensor 44P can be representative of rotational position or movement of the intralumenal device 20. Total voltage readings between 0.0 V and 6.0 V are representative of the degree of linear and rotational position and movement.

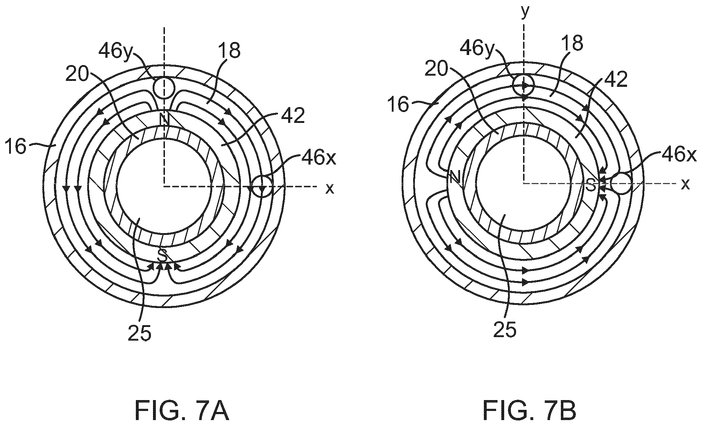

In some embodiments, with reference to **FIG. 7A** and **FIG. 7B****,** a pathway 18 of a control handle 16 has two orthogonal (or off angle) arrays of sensors 46x and 46y and an intralumenal device 20 extending therethrough has a diametrically magnetized member (or ring magnet) 42 with its magnetic field lines being diametrical to the intralumenal device 20 or perpendicular to the longitudinal axis of the intralumenal device 20. The emitter member 42 is configured to produce a voltage change in the sensors of both arrays, where a purely linear movement without rotational movement of the intralumenal device 20 produces generally identical responsive signals between each sensor 46x of the orthogonal arrays and generally identical responsive signals between each sensor 46y of the orthogonal arrays, and a purely rotational movement of the intralumenal device 20 without linear movement produces responsive signals in only the orthogonal pair of 46x and 46y in the same axial position. A combination of linear and rotational movement in the intralumenal device 20 produces responsive signals in the sensors of both arrays that are representative of the combination movement.

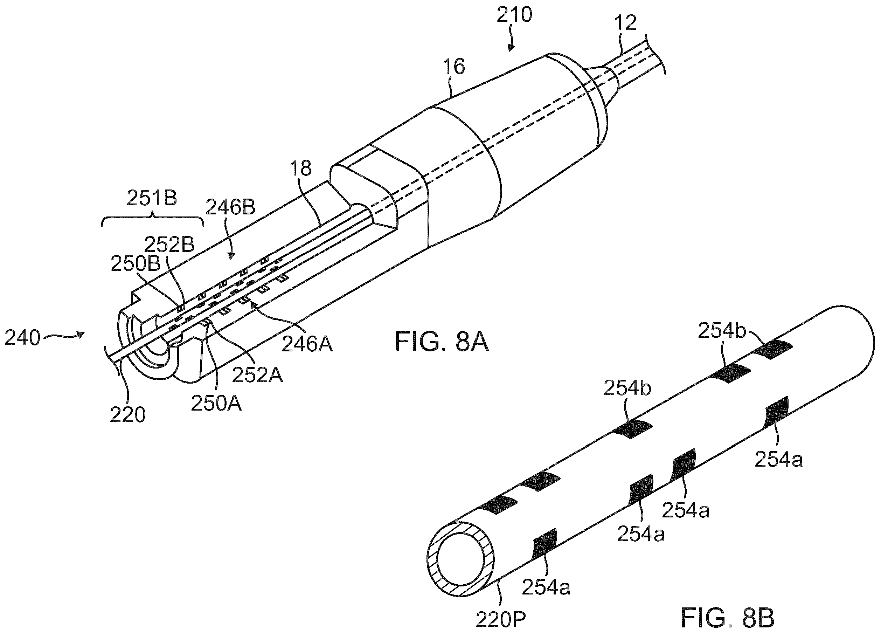

In some embodiments, with reference to **FIG. 8A and FIG. 8B****,** the components of a position sensing assembly 240 include an optical sensor 252 and an optical emitter (or sensor marker), including, for example, an optically-detectable pattern 254 whose optical emission when illuminated by an optical source 250 is detected by the optical sensor 252. The optical source 250 and the optical sensor 252 may form an optical unit in the control handle 16. The optically-detectable pattern 254 is provided on a proximal portion 220P of an intralumenal device 220, and the optical source 250 and the optical sensor 252 are situated along a pathway 218 of a control handle 216 of a guiding sheath 210 that receives the intralumenal device 220. The optical sensor 252 generates signals in response to the presence, movement and location of the illuminated optically-detectable pattern 254, wherein such generated signals are representative of the displacement and position (linear and/or rotational) of the proximal portion 220P of the intralumenal device 220 relative to the control handle 16, if not also of the distal portion of the intralumenal device relative to the control handle 16.

In some embodiments, a plurality of optical units are provided to form a linear optical array that extends longitudinally along the pathway 18. In some embodiments, two optical arrays 246a and 246b are provided, diametrically opposing each other along the pathway 18, so as to minimize interference between optical illumination and sensing between the two arrays.

As shown in **FIG. 8B****,** the optically-detectable pattern may include at least two different patterns 254a and 254b provided on different or opposing locations on the surface of the intralumenal device 220 so that the signals generated by the optical sensor 252 in response to the two different patterns can indicate angular rotation of the intralumenal device 220 about its longitudinal axis. The optically-detectable patterns can also be unique to an individual intralumenal device so that each intralumenal device can be identified and limited as a single-use device.

In some embodiments, one or more of the intralumenal devices 24, 26 includes an identification marker 170 and the control handle 16 of the guiding sheath 10 includes an identification circuit 172, as shown in **FIG. 2A****.** The identification circuit 172 is configured to determine or identify the predetermined configuration and geometry of the intralumenal device based at least in part on the identification marker 170, which may include a radio frequency identification (RFID) circuit. The identification marker 170 may be affixed to a proximal portion of each intralumenal device so that it is read upon insertion into the control handle or shortly after by the identification circuit 172 situated along the pathway 18 of the control handle 16. In some embodiments, the identification marker 170 may be read by a scan unit 176 separate from or in addition to the identification circuit 172 in the control handle 16, and in communication with an ID subroutine 159 of the position sensing module 160 **(****FIG. 4B****).**

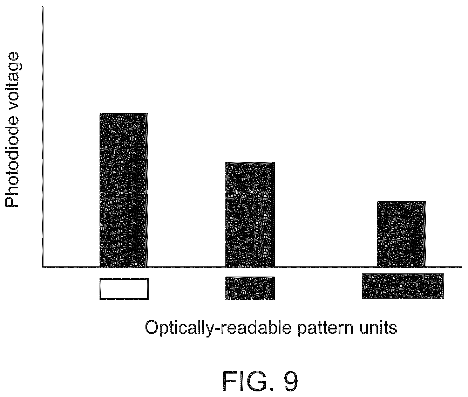

In some embodiments, the optical source 250 includes an LED, the optical sensor 252 includes a photodiode, and the optically-detectable pattern 254 as the optical emitter includes a bar code or binary-alternating symbols on multi-axial locations circumferentially on the surface of the intralumenal device 220. It is understood that the optically-detectable pattern, as an optical emitter, typically comprising of discrete indicia of dark and light shades reflects light energy in a corresponding pattern that is detected by the optical detector whose output voltage increases with higher reflected light energy and decreases with lower reflected light energy, as shown in **FIG. 9****.** The position sensing assembly 240 may include multiple optical sources 250 and multiple optical detectors 252 arranged in pairs, each pair located at a different location along the pathway 18. It is understood that parameters of the configuration of position sensing assembly 240 depend on a variety of factors, including the size of the field of illumination of each emitter, the length of the optically-detectable pattern, the acceptable degree of uncertainty in the exact location of the intralumenal device relative to the guiding sheath, etc.

It bears emphasis that all of the components of the position sensing assembly are within the confines of the control handle of the guiding sheath, which is well outside of the patient's heart, if not outside the patient's body for improved safety to the patient. Moreover, electrical connections to and from the electromagnetic sensors do not extend distally past the control handle. That is, electrical connectors such as lead wires for transmitting signals representative of the position of the distal tip of either the first or second intralumenal device no longer need to pass through the entire length of the shafts of these instruments, which saves significantly on the manual labor that would otherwise be necessary for construction and assembly of these instruments, not to mention the space saved in the distal tips that would otherwise be occupied by conventional electromagnetic position sensors using x/y/z coils.

It is understood that means for determining position, including means for determining position of the distal portion of an intralumenal device, are shown and described in one of the many examples in relation to the magnetic field emitters and magnetic field sensors, including, for example, the magnets and the Hall-effect sensors, shown in **FIG. 2A** and **FIG. 2B****,** and in relation to the optical sensors and the optical emitters illuminated by the optical sources, including, for example, the photodiodes, the optically-readable patterns, and the LEDs, shown in **FIG. 8A** and **FIG. 8B****.** It is further understood that determining position may include determining length, and thus means for determining position may be used interchangeably with means for determining length, as needed or appropriate. It is further understood that means for determining position, include conductive members configured to complete an electrical circuit and sensors for detecting a completed electrical circuit and sensors for measuring impedance, as discussed further below.

It is also understood that the position sensing assembly is not limited to only electromagnetic components or optical components, and further that both of these types of components may be incorporated and used together as needed or desired.

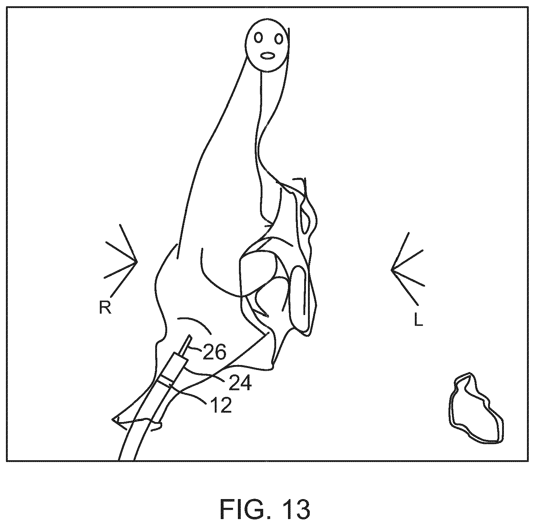

With reference to **FIG. 13****,** an operator has acquired a 3D map of a patient body, including the patient's heart by means of the advanced electrophysiology (EP) imaging system S, such as the CARTO^{®} 3 System. The image generated by the system S that is displayed not only includes the 3D map of the patient's heart, but it also includes depictions of a first (or outer) intralumenal device 24, for example, a dilator, and a second (or inner) intralumenal device 26, for example, a transseptal needle, especially their respective distal ends.

In arriving at this image of the first and second intralumenal devices according to some embodiments, the user has taken the following actions, including the user using the pointing device and the graphical user interface to enter various user-selectable parameters of the procedure into the system S, including the selection of the guiding sheath and the first and second intralumenal devices to be used in the procedure. Geometries and configurations of guiding sheath 10 and of intralumenal devices suitable for use with the system S, including the selected first and second intralumenal devices 24 and 26, have been stored in the configurations and geometries subroutine 156 of the memory 134 **(****FIG. 4B****).** The configurations and geometries provide predictable and reliable determination of the location, position and/or orientation of the distal portion of an intralumenal device based on the location, position and/or orientation of the proximal portion of the intralumenal device in relation to the control handle of a guiding sheath through which intralumenal device extends. With reference to **FIG. 3**, parameters stored in the configurations and geometries subroutine 156 may include, for example, the following:

**Table 1**

| Parameters | Definition |
|---|---|
| LT | total length of guiding sheath 10 (including sheath 12, control handle 16 and hemostatis valve 22) |
| LS1 | distance between sensor 44 and distal end 12D of guiding sheath 10 |
| LIN1 | insertion length of first intralumenal device 24 (= length of shaft 28 of first intralumenal device 24) |
| LM1 (=LS1 -AD1) | distance between proximal emitter 42A1 of first intralumenal device 24 and distal end 12D |
| LM2 (=LS1) | distance between middle emitter 42A2 of first intralumenal device 24 and distal end 12D |
| LM3 (= LS1 + DD1) | distance between distal emitter 42A3 of first intralumenal device 24 and distal end 12D |
| AD1 | approach distance of intralumenal device 24 to distal end 12D of guiding sheath 10 |
| DD1 | length of exposed (deployed) distal portion of first intralumenal device 24 distal of distal end 12D when device 24 is deployed |
| LIN2 | insertion length of second intralumenal device 26 (= length of elongated body 31 of second intralumenal device 26) |
| LE1 (=LS1 + DD1 - AD2) | distance between proximal emitter 42B1 of second intralumenal device 26 and distal end 28D |
| LE2(=LS1 + DD1) | distance between middle emitter 42B2 of second intralumenal device 26 and distal end 28D |
| LE3 (=LS1+ DD1 + DD2) | distance between distal emitter 42B3 of second intralumenal device 26 and distal end 28D |
| AD2 | approach distance of intralumenal device 26 to distal end 28D of first intralumenal device 24 |
| DD2 | Length of exposed (deployed) distal portion of second intralumenal device 26 distal of distal end 28D when device 26 is deployed |

With the guiding sheath and the first and second intralumenal devices to be used in a procedure identified by the user, the system can retrieve from the configuration and geometries subroutine 156 the relevant parameters, including the above-listed parameters, to be used and applied by the position sensing module 160.

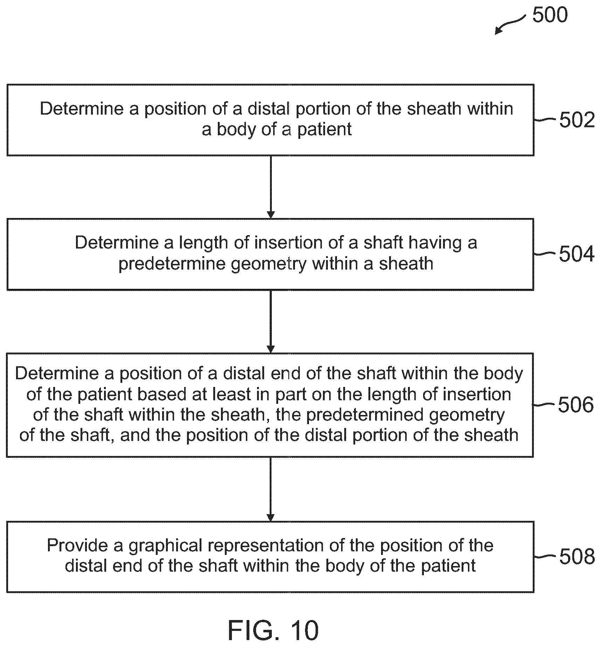

In some embodiments of the system, devices and related methods, reference is made to the flow chart of **FIG. 10****.** In Block 502, a determination is made of the position of a distal portion of the guiding sheath within a body of a patient, for example, by the mapping module 153. In Block 504, a determination is made of a length of insertion of a shaft of an intralumenal device having a predetermined geometry within the sheath. In Block 506, a determination is made of a position of the distal end of the shaft within the body of the patient based at least in part on the length of insertion of the shaft within the sheath. In Block 508, a graphical representation is provided of the position of the distal end of the shaft within the body of the patient.

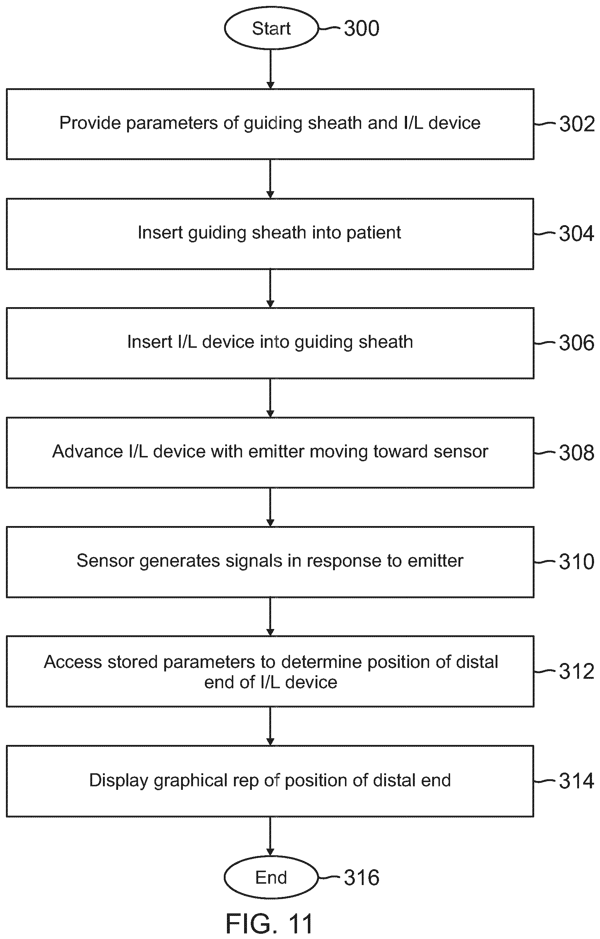

In some embodiments of methods related to the system and devices, reference is made to the flow chart of **FIG. 11****.** The flow chart begins at Block 300. In Block 302, parameters of the configuration and geometries of a guiding sheath and an intralumenal device are determined and stored in the configurations/geometries subroutine of the memory of the EP imaging system S. In Block 304, the guiding sheath is inserted into the patient. In Block 304, the intralumenal device is inserted into the guiding sheath. In Block 306, the intralumenal device is advanced relative to the guiding sheath with an emitter on the intralumenal device moving toward a sensor situated in a control handle of the guiding sheath. In Block 308, the sensor senses the emitter and generates signals in response to the emitter. In Block 310, the position sensing module accesses the parameters and determines the position of the distal end of the intralumenal device based on the signals. In Block 312, the EP imaging system S displays the position of the distal end of the intralumenal device. It is understood that position may include location and orientation.

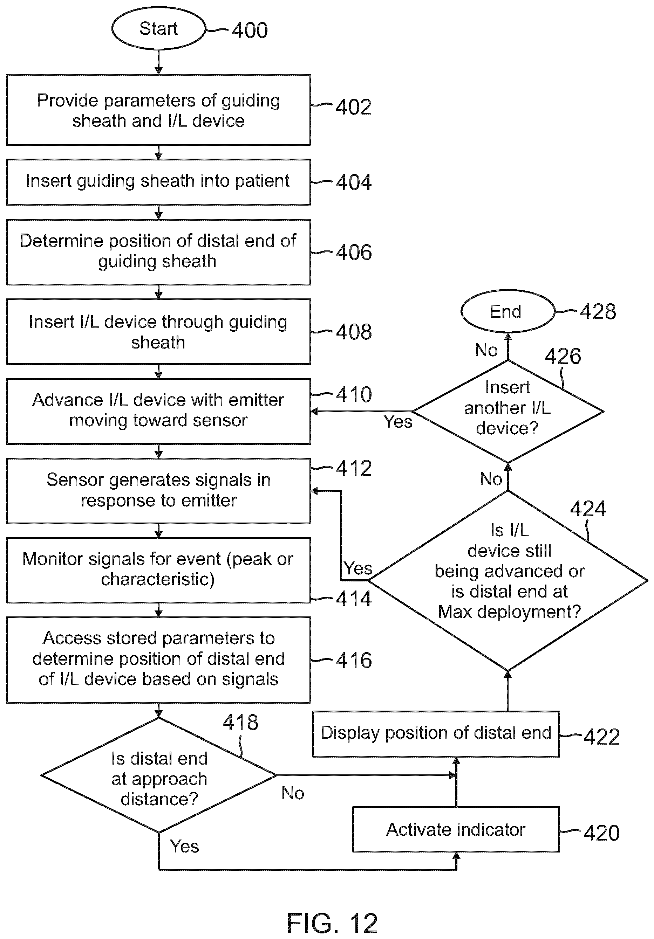

In some embodiments of the methods related to the system and device, reference is made to the flow chart of **FIG. 12****.** The flow chart begins with Block 400. In Block 402, the parameters of Table 1, are determined and stored in Configurations/Geometries Subroutine of Memory 134. In Block 404, the user inserts the guiding sheath 10 into the patient's vasculature, e.g., a femoral artery, and secures the position of the guiding sheath 10 in fixed relationship with the patient. In Block 406, the EP imaging system S determines the position of the distal end 12D of the guiding sheath by, for example, an electromagnetic navigation sensor, as known in the art. In Block 408, the user inserts the first intralumenal device 24 into the lumen 14 of the guiding sheath 10 via the hemostatis valve 22. In Block 410, the user advances the device 24 distally toward the distal end 12D of the guiding sheath 10. In Block 412, as distal emitter 42A1 on the shaft 28 of the first intralumenal device 24 approaches the sensor 44 in the control handle 16 of the guiding sheath, the sensor 44 senses the distal emitter 42A1 and generates signals in response to the emitter 42A1. In Block 414, the position sensing module 160 monitors the signals for an event, for example, a peak (a calibrated maximum) or a particular characteristic, as representative of the distal emitter 42A1 being in closest proximity to the sensor 44, in side-by-side alignment therewith, along the longitudinal axis of the control handle 16. In Block 416, the position sensing module 160 accesses the parameters stored in the configurations and geometries subroutine 156 based on the signal event. Because the first intralumenal device 24 has a predetermined geometry, for example, including that the position LM1 of the distal emitter 42A1 on the shaft 28 relative to the distal end 28D is equal to the length LS1 (namely, the distance between the sensor 44 and the distal end 12D of the guiding sheath) less the approach distance AD1, the signals generated by the sensor 44 when the sensor 44 and the distal emitter 42A1 are in their closest proximity, in side-by-side alignment, advantageously indicate a relative position between the device 24 and the guiding sheath 10, including that the distal end 28D of the first intralumenal device 24 has reached the approach distance AD1 of the distal end 12D of the guiding sheath. At Decision Block 418, a query is made as to whether the event indicates or is related to the distal end 28D reaching the approach distance AD1. If the sensor 44 is generating signals (or a peak signal) in response to the distal emitter 42A1, then the answer to the query is yes and the flow chart continues to Block 420 which activates an indicator, e.g., a visual alarm 160 and/or an audio alarm 162 to notify the user that the distal end 28D has reached the approach distance AD1 and that the distal end 28D may soon protrude from the distal end 12D of the sheath 12, before continuing to Block 422. The indicator may automatically terminate after a predetermined duration or remain active until deactivated by the user. If the sensor 44 is not generating signals (or a peak signal) in response to the distal emitter 42A1, then the answer to the query of Block 418 is no and the flow chart continues to Block 422 where the processing unit 132 displays (or continues to display) the distal end 28D on the display monitor in real time according to the parameters of the configurations and geometries of the first intralumenal device 24 relative to the display of the distal end 12D of guiding sheath within the patient's heart on the display monitor. In this manner, the determined position and display of the distal end 28D of the intralumenal device 24 are advantageously based on signals generated by the proximal end of the first intralumenal device 24.

At Decision Block 424, a query is made as to whether the first intralumenal device 24 is continuing in its advancement distally relative to the guiding sheath 10. This determination can be made by, for example, assessing changes in the signals sensed by the sensor 44 with reference to the parameters of the first intralumenal device 24. If yes, the flow chart returns to Block 412. As the mid-emitter 42A2 of the first intralumenal device 24 approaches the sensor 44, the sensor 44 senses the mid-emitter 42A2 and generates signals in response to the mid-emitter 42A2. In Block 414, the positioning module 160 monitors the signals for an event, for example, a peak (a calibrated maximum) or a particular characteristic, as representative of the mid-emmitter 42A2 being in closest proximity to the sensor 44, in side-by-side alignment therewith, along the longitudinal axis of the control handle 16. In Block 416, the position sensing module 160 accesses the parameters stored in the configurations and geometries subroutine 156 for the first intralumenal device 24 and the mid-emitter 42A2 based on the signal event. Because the first intralumenal device 24 has a predetermined geometry, for example, including that the position of the mid-emitter 42A2 on the shaft 28 relative to the distal end 28D is equal to the length LS1, the signals generated by the sensor 44 when it and the mid-emitter 42A2 are in their closest proximity, in side-by-side alignment, advantageously indicate a relative position between the device 24 and the guiding sheath 10, including that the distal end 28D is at the distal end 12D. As the distal end 28D is not at the Approach Distance AD1 in Block 418 (but has in fact moved past it), the flow chart continues to Block 422 where the processing unit 132 continues to display on the display monitor in real time the location of the distal end 28D relative to the distal end 12D of guiding sheath within the patient's heart on the display monitor. Again in this manner, the determined position and display of the distal end 28D of the intralumenal device 24 are advantageously based on signals generated by the proximal end of the first intralumenal device. At Decision Block 424, a query is made as to whether the first intralumenal device 24 is continuing in its advancement distally relative to the guiding sheath 10. This determination can be made by, for example, assessing changes in the signals sensed by the sensor 44 with reference to the parameters of the first intralumenal device 24 or by input by the user that he is still advancing the first intralumenal device 24. If yes, the flow chart returns to Block 412. Additionally or in lieu, the query of Decision Block 424 may include a determination as to whether the distal end 28D is at maximum deployment distance DD1 by accessing the parameters stored in the configurations and geometries subroutine 146. If no, the flow chart returns to Block 412.

The loop including Blocks 412, 414, 416, 418, 420, 422 and 424 can be repeated for the proximal emitter 42A3 on the first intralumenal device 24. In Block 416, the position sensing module 160 accesses the parameters stored in the configuration and geometries subroutine 156 for the first intralumenal device 24 and the proximal emitter 42A3. Because the first intralumenal device 24 has a predetermined geometry, for example, including that the position of the proximal emitter 42A3 on the shaft 28 relative to the distal end 28D is equal to the length LS1 plus the calibrated maximum exposed distance DD1 of the distal end 28D of the first intralumenal device 24 distal of the distal end 12D of the control handle, the signals generated when the sensor 44 and the proximal emitter 42A3 are in their closest proximity to, in side-by-side alignment , advantageously indicate a relative position between the device 24 and the guiding sheath 10, including that the distal end 28D is at the calibrated maximum exposed length DD1 distal of distal end 12D. Again, because the distal end 28D has advanced past the approach distance AD1, the flow chart continues to Block 422. In Block 422, the processing unit 132 continues to display on the display monitor in real time the location of the distal end 28D relative to the distal end 12D of guiding sheath within the patient's heart on the display monitor, where the determined position and display of the distal end 28D are advantageously based on signals generated by the proximal end of the first intralumenal device 24.

When the first intralumenal device is no longer being advanced relative to the guiding sheath as determined by the position sensing module assessing changes in the signals sensed by the sensor 44 with reference to the parameters of the first intralumenal device 24 or by input by the user that he is still no longer advancing the first intralumenal device 24, the Decision Block 424 continues to Decision Block 426 with a query as to whether another intralumenal device is to be inserted. This determination may be based on user input. If yes, the flow chart returns to Block 410 where the second intralumenal device 26 is inserted into the first intralumenal device 24 via the valve 23, and advanced distally toward the distal end 28D of the first intralumenal device 24. In Block 412, as distal emitter 42B1 on the elongated body 31 of the second intralumenal device 26 approaches the sensor 44 in the control handle of the guiding sheath, the sensor 44 sensing the emitter 42B1 generates signals in response to the emitter 42B1. In Block 414, the position sensing module 160 monitors the signals for an event, for example, a peak (a calibrated maximum) or a particular characteristic, as representative of the sensor 44 and the distal emitter 42B1 in closest proximity, in side-by-side in alignment, along the longitudinal axis of the control handle 16. In Block 416, the position sensing module 160 accesses the parameters stored in the configurations and geometries subroutine 156 based on the signal event. Because the second intralumenal device 26 has a predetermined geometry, for example, including that the position LE1 of the distal emitter 42B1 on the elongated body 31 relative to the distal end 26D is equal to the length LS1 plus the calibrated maximum exposed distance DD1 of the first intralumenal device 24 less the approach distance AD2, the signals generated when the advantageously indicate a relative position between the device 24 and the guiding sheath 10, including that the distal end 26D of the second intralumenal device 26 is at the approach distance AD2 of the distal end 28D of the first intralumenal device 24. In this manner, the determined position and display of the distal end 26D of the second intralumenal device 26 are advantageously based on signals generated by the proximal end of the second intralumenal device 26.

In Decision Block 418, the processing unit 132 in response to the signals representative of the distal end 26D being at the approach distance AD2 of the distal end 28D activates the indicator (e.g., the audio alarm 162 or the visual alarm 164) to notify the user that the distal end 26D has reached the approach distance AD2 and that the distal end 26D may soon protrude from the distal end 28D of the first intralumenal device 24. In Block 422, the processing unit 132 begins to display the location of the distal end 26D on the display monitor 127 in real time according to the parameters of the configuration and geometry of the second intralumenal device 26, relative to the display of the distal end 28D and the distal end 12D within the patient's heart on the display monitor.

In Decision Block 424, if the user continues to advance the second intralumenal device 26 distally relative to the guiding sheath 10, the flow chart returns to Block 412. The loop including Blocks 412, 414, 416, 418, 422 and 424 can be repeated for the mid emitter 42B2 and the proximal emitter 42B3. For mid emitter 42B2, in Block 414, the position sensing module 160 monitors the signals for an event, for example, a peak (a calibrated maximum) or a particular characteristic, as representative of the sensor 44 and the mid-emitter 42B2 being in closest proximity, in side-by-side alignment, along the longitudinal axis of the control handle 16. In Block 416, the position sensing module 160 accesses the parameters stored in the configurations and geometries subroutine 156 based on the signal event. Because the second intralumenal device 26 has a predetermined geometry including that the position of the mid emitter 42B2 on the elongated body 31 relative to the distal end 26D is equal to the length LS1 plus the calibrated maximum exposed length DD1 of the first intralumenal device 24, the signals generated when the the sensor 44 and the mid-emitter 42B2 are in closest proximity, in side-by-side alignment, advantageously indicate a relative position between the device 24 and the guiding sheath 10, including that the distal end 26D has reached the distal end 28D. In Block 422, the processing unit 132 continues to display on the display monitor in real time the location of the distal end 26D of the second intralumenal device 26 relative to the distal end 28D of the first intralumenal device 24 and the distal end 12D of the guiding sheath 10 within the patient's heart on the display monitor.

For proximal emitter 42B3, in Block 414, the position sensing module 160 monitors the signals for an event, for example, a peak (a calibrated maximum) or a particular characteristic, as representative of the sensor 44 and the proximal emitter 42B3 in closest proximity, in side-by-side alignment, along the longitudinal axis of the control handle 16. In Block 416, the position sensing module 160 accesses the parameters stored in the configurations and geometries subroutine 156 based on the signal event. Because the second intralumenal device 26 has a predetermined geometry of the second intralumenal device 26, for example, including that the position of the proximal emitter 42B3 on the elongated body 31 relative to the distal end 26D is equal to the length LS1 plus the calibrated maximum exposed distance DD1 and the calibrated maximum exposed distance DD2, the signals generated when the sensor 44 and the proximal emitter 42B3 are in closest proximity, in side-by-side alignment, advantageously indicate a relative position between the device 24 and the guiding sheath 10, including that the distal end 26D is at the calibrated maximum exposed length distal of distal end 28D. In Block 422, the processing unit 132 continues to display on the display monitor in real time the location of the distal end 26D and the distal end 28D relative to the distal end 12D of guiding sheath within the patient's heart on the display monitor.

It is understood that the detection of any emitter on the intralumenal devices, for example, 42A1, 42A2, 42A3, 42B1, 42B2. 42B3, by the sensor gives rise to signals that are representative of a length of insertion of the intralumenal device whose emitter is being detected because of the predetermined configurations and geometries of the intralumenal device, including, for example, the parameters LM1, LM2, LM3, LE1, LE2, LE3. And further because of the predetermined configurations and geometries of the intralumenal device, a length of insertion can be used to determine position (or at least the location) of a distal end of the intralumenal device relative to the guiding sheath.

It is further understood that the flow chart accommodates any plurality of emitters so provided the intralumenal device is being advanced relative to the control handle. That is, the loop repeats for each emitter until the Decision Block 424 produces a "no" to the query of whether the intralumenal device is still being advanced relative to the control handle. And, if no other intralumenal device is to be inserted per Decision Block 426, the flow chart may end at Block 428.

It is understood that in some embodiments where one or more of the emitters 42A1, 42A2, 42A3, 42B1, 42B2 and 42B3 are off-axis on the shaft 28 of the first intralumenal device 24 and the elongated body 31 of the second intralumenal device 26, respectively, the signals generated by the sensor 44 in response thereto are also representative of rotation of the first and second intralumenal devices 24 and 26 about their respective longitudinal axes, as shown in **FIG. 5A, FIG. 5B** and **FIG. 5C****.** Parameters include calibrated sensor readings that correspond with rotational angles of the off-axis emitters. Thus, in Block 312 and Block 416, the parameters accessed include signals values or characteristics that are representative of the position of the distal ends 28D and 26D including location and rotational orientation. In that regard, the predetermined configurations and geometries of the first and second intraluminal devices include predetermined rigidity of the devices over their length and their torsional rigidity such that the application of a translation and/or rotational force at the proximal ends of the devices produces a predictable translation and/or rotation at their distal ends.

In some embodiments, the position sensing module 160 includes a table of calibrations of signal readings corresponding to the longitudinal and rotational positions of each emitter of the first and second intralumenal devices relative to the sensors 44 that may be referenced by the module to determine and/or confirm the relative positions of the emitters on the proximal portion of the devices 24 and 26 to the sensor 44, which are referenced by the processing unit to determine (or reliably predict) the positions of distal ends 28D and 26D of the intralumenal devices 24 and 26 for display on the display monitor.

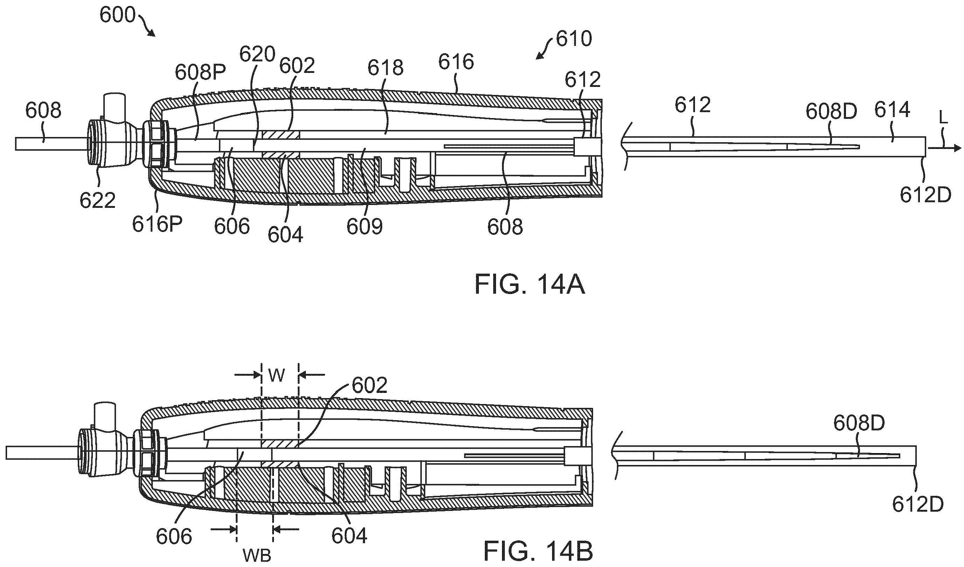

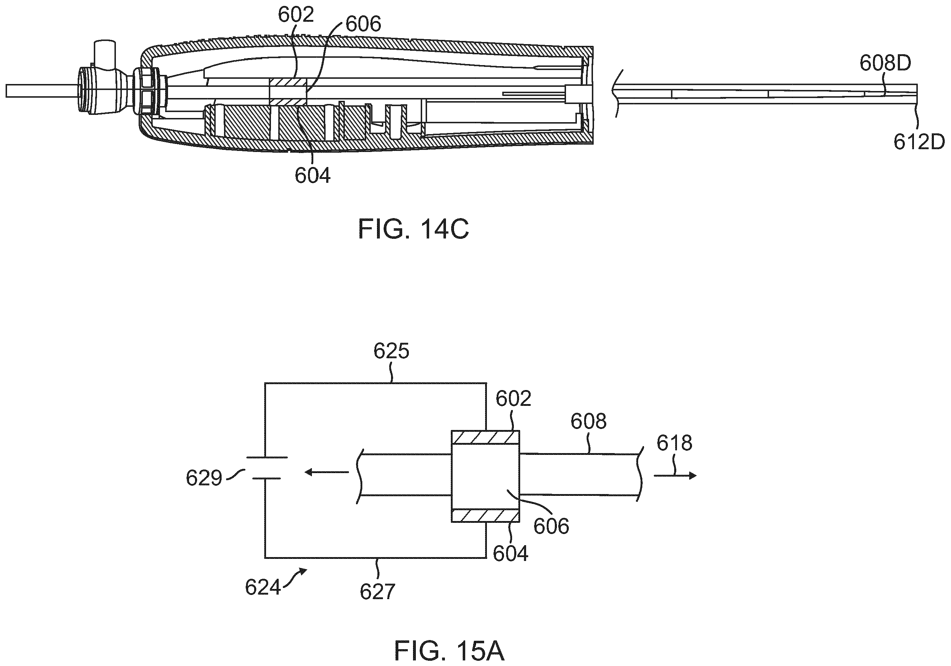

With reference to **FIG. 14A, FIG. 14B** and **FIG. 14C****,** in some embodiments, the position sensing assembly 600 includes first and second electrically-conductive members (or electrodes) 602, 604 situated along a pathway 618 of a control handle 616 of a guiding sheath 610, and a third electrically-conductive member (or electrode) 606 situated on a proximal portion 608P of an intralumenal device 608 configured for insertion into the pathway 618 and distal advancement through a lumen 614 of a sheath 612 extending distally of the control handle 616. The first and second circuit members 602, 604 are separate and distinct from each other and may be disposed, for example, across the pathway 618 from each other, in diametrically opposite positions, and each may have a same dimension or width W along the pathway 618 which is aligned with a longitudinal axis L defined by the control handle 616. The third conductive member 606 may be configured as a circumferential band 620 disposed on an outer surface 609 of the intralumenal device 608. The band has a width WB along the longitudinal axis that is equal to or greater than the width W of the first and second.

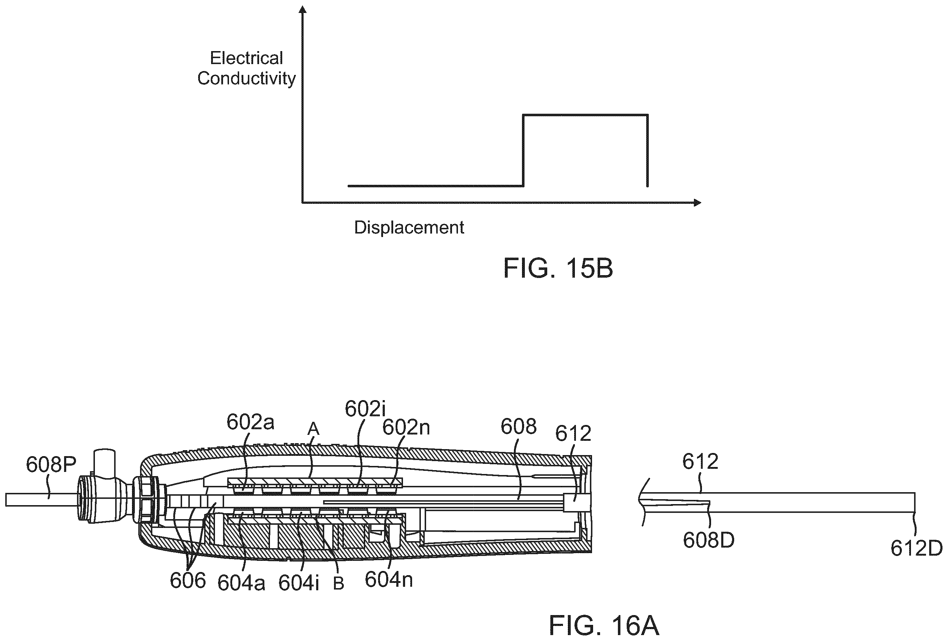

In use, a distal end 608D of the intralumenal device 608 is inserted into a hemostatis valve 622 at a proximal end 616P of the control handle 616 to enter the pathway 618. The distal end 608D then enters the lumen 614 of the sheath 612 of the guiding sheath 610. As the advancement of the intralumenal device 608 continues distally, the proximal portion 608P enters the pathway 618 and the third conductive member 606 approaches the first and second conductive members 602, 604 **(****FIG. 14A****).** With further distal advancement, the third conductive member 606 comes into contact with both of the first and second conductive members 602, 604 **(****FIG. 14B****).** As shown in **FIG. 15****,** each of the first and second conductive elements 602, 604 is connected to a respective lead wire 625, 627 that extends through an electrical connector (see electrical connector 17 in **FIG. 1A****)** which enables connection to a power/current source 629. Thus, by establishing contact with the first and second conductive members 602, 604, the third conductive element 606 completes an electrical circuit 624 that includes the first, third and second conductive members whereupon electrical conductivity is detected **(****FIG. 15B****);** that is, once the third conductive member 606 is in contact with the first and second conductive members 602, 604, the circuit 624 is completed thereby identifying the presence of the intralumenal device 608. Because the device 608 has a predetermined geometry, e.g., a predetermined position of the third conductive member 606 from the distal or proximal end of the device 608, the signals generated when the electrical circuit 624 is completed by conductive contact between the first, second and third conductive members 602, 604, 606 advantageously indicate a relative position between the device 608 and the guiding sheath 610, for example, including that the distal end 608D is approaching or protruding from a distal end 612D of the sheath 612. As shown in **FIG. 15B****,** the electrical conductivity remains long as there is contact between the members 602 and 606 and between members 606 and 604 during linear translation of the device 608 relative to the control handle 616.

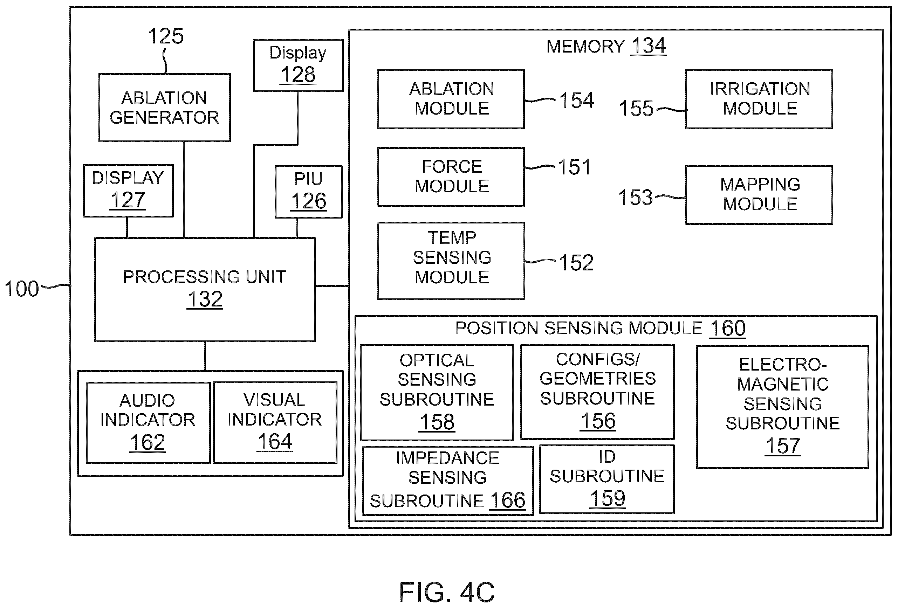

In that regard, a system S as shown in **FIG. 4C****,** includes a circuit sensing subroutine 165 in a position sensing module 160. By measuring and monitoring the completion of the circuit 624, the system S is configured to determine the position of the proximal portion of the device 608 relative to the control handle 616 and thus the position of the distal end 608D relative to the distal end of the sheath 612 based on the configurations and geometries subroutine 156 of the memory 134 for the device 608. The stored configuration and geometry of the device 608 provides predictable and reliable determination of the location, position and/or orientation of the distal end 608D of the device 608 based on the location, position and/or orientation of the proximal portion 608P of the device 608 in relation to the control handle 616 of the guiding sheath 610 through which the device 608 extends.

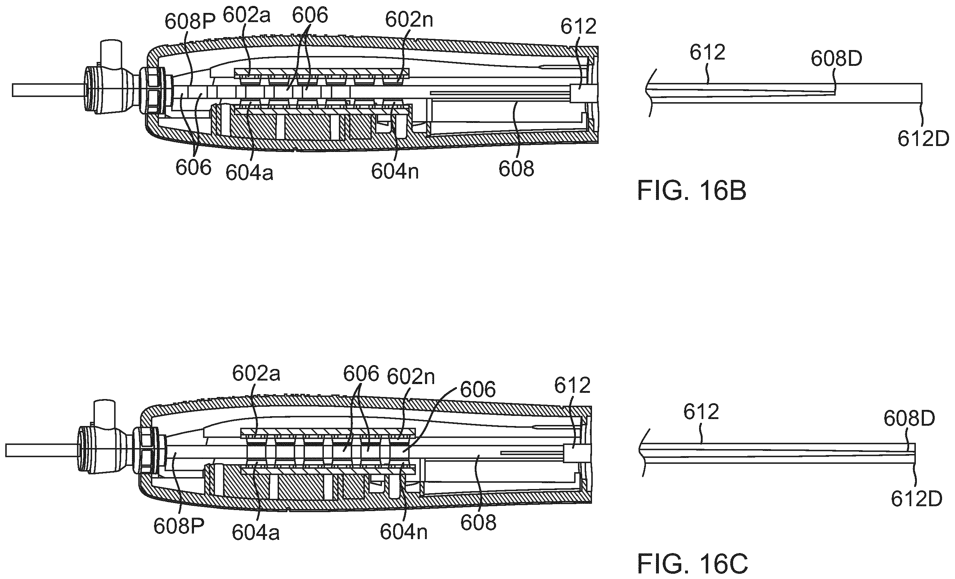

With reference to **FIG. 16A****,** **FIG. 16B** and **FIG. 16C****,** in some embodiments, the control handle 616 includes a first array A of a plurality of first conductive members 602 and a second array B of a common plurality of second conductive members 604, with each second conductive member being diametrically opposed from a respective first conductive member along the pathway 618 to form a respective pair of separate and distinct conductive members. A third array C of a plurality of third conductive members 606 is situated on the proximal portion 608P of the intralumenal device 608 where each of third conductive member 606 can contact a pair of conductive members 602, 604 in completing a respective electrical circuit 624 with the connected pairs 602, 604 with movement of the device 608 relative to the control handle 616,

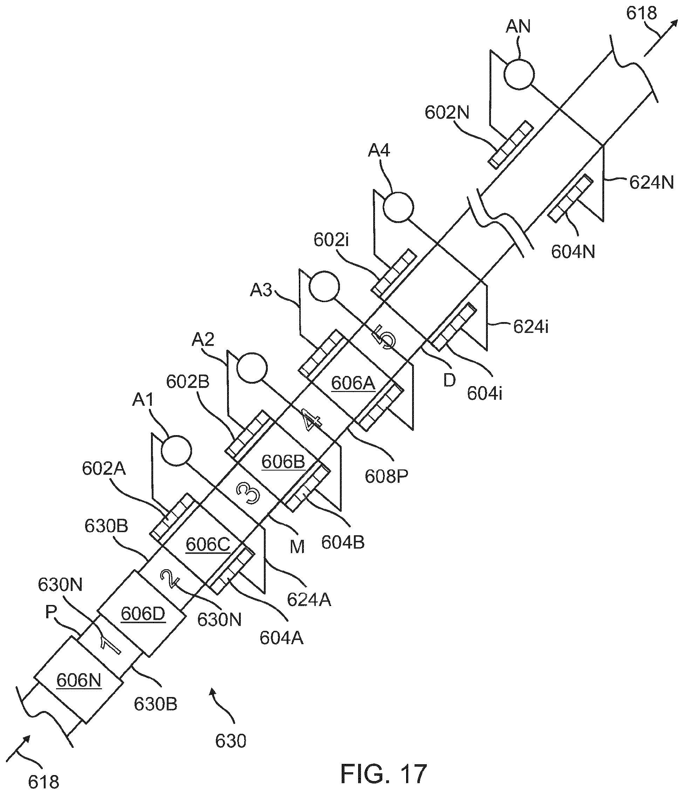

As shown in **FIG. 17****,** as the intralumenal device 608 is advanced distally in the control handle 616 along the pathway 618 and the third array C of third conductive members 608 approaches the first and second arrays A, B, the number of circuits completed between a respective pair of first and second conductive members 602, 604 by the third conductive members 606 indicates relative position and movement between the proximal portion 608P of the device 608 and the control handle 616, and thus relative position and movement between the distal end 608D of the device and a distal end 610D of the guiding sheath 610 based on the configuration and geometry of the device 608.

With an N plurality of first conductive members 602 and second conductive members 604, and an N plurality of current sensors A1-AN, the completion of each circuit between a respective pair of first and second conductive members 602, 604 by a connecting third conductive member 606 is detected by current sensors A1-AN. By the progression of current sensors A1-AN detecting the completion of the circuits along the arrays A, B, the system S determines the relative position and movement of the proximal portion 608P of the device 608 and the control handle 616. The detection of the completion of circuits A1, indicates the proximal portion 608P is in position P1 relative to the control handle. For example, as shown in **FIG. 17****,** the detection of the completion of circuits A1, A2, A3 by third conductive members 606A, 606B, 606C indicates that the proximal portion 608P is in a respective position relative to the control handle 616. Likewise, it is understood that the detection of the completion of circuits A1, A2, A3, A4 by third conductive members 606A, 606B, 606C, 606D indicates that the proximal portion 608P is in a more distal respective position relative to the control handle 616. Each respective position relative to the control handle is determinative of a respective position of the distal end 608D of the device 608 relative to the distal end of the guiding sheath 610 based on known configurations and geometries of the device 608 and the guiding sheath 610. It is understood that the width of and spacing between the conductive members of the arrays A1, A2, A3 can vary depending upon the desired accuracy or resolution of the relative position and movement of the device 608. In some embodiments, the third conductive members 606 may be configured as ring electrodes or mounted flexible electrodes.

In some embodiments, as shown in **FIG. 17****,** the proximal portion 608P of the device 608 includes visual indicia 630 configured for viewing by the user in visually notifying the user of the relative position between the device 608 and the control handle 616, and thus the relative position between the distal end 608D of the device 608 and the distal end of the sheath 612 of the guiding sheath 610. In some embodiments, the visual indicia 630 may include, for example, alphanumeric symbols 630N at selected locations indicating the distances from the respective locations to a proximal end of the device 608, wherein the locations are in between adjacent third conductive members 606. In some embodiments, the visual indicia 630 may include color-coded marker or bands 630B, for example, a green marker at a distal location D, a yellow marker at a mid-location M and a red marker at a proximal location P, wherein the green marker indicates that the distal end 608D of the device 608 is proximal of the distal end 612D of the sheath 612, the yellow marker indicates that the distal end 608D is approaching the distal end 612, and the red marker indicates that the distal end 608D will soon pass and protrude from the distal end 612 with continued distal advancement.

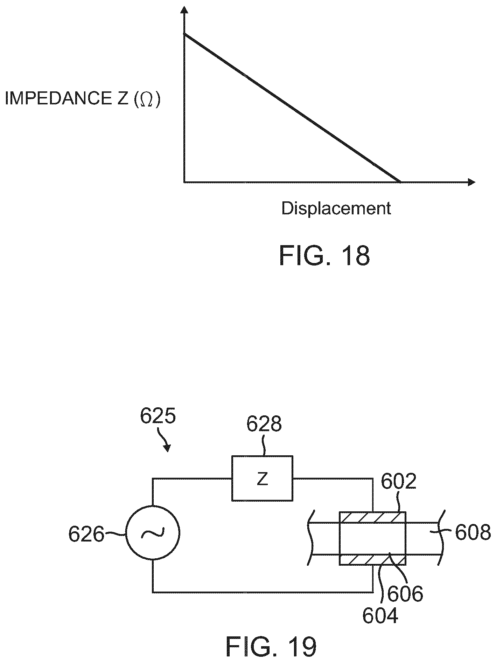

It is understood that electrical impedance decreases when the area of cross-section of a conductor is increased, as shown in **FIG. 18****.** As the device 608 is advanced (or displaced) distally relative to the control handle 616, the contact area between the third conductive element 606 and the first and second conductive elements 602, 604 increases which results in a decreasing electrical impedance Z of the completed circuit. Accordingly, when calibrated to a positional value, the impedance reading of the completed circuit indicates a linear position of the device 608 relative to the control handle 616 and thus relative to the guiding sheath 610 and the distal end 612D of the sheath 612. The positional value can be correlated to a distance that the distal end 608D of the device 608 protrudes from the distal end 612D of the sheath 612, or an approach distance remaining before the distal tip 608D exits the distal end 612D of the sheath 612.

In some embodiments, as shown in **FIG. 19****,** a circuit 625 completed by the first, second and third conductive members 602, 604, 606 includes a signal generator 626 and an impedance measuring device 628, e.g., an oscilloscope, an AC volt meter or an impedance meter. FIG. 18 is a graph of measured impedance (in Ohms) across the circuit 625 as a function of the distance (displacement) of the third conductive member 606 relative to the first and second conductive members 602, 604, with represents the amount of contact surface area between the first, second and conductive members 602, 604, 606. When the third conductive member 606 has not made contact with the first and second conductive members 602, 604 **(****FIG. 14A****),** the graph of FIG. 18 shows the measured impedance at a maximum. As the intralumenal device 608 is advanced distally, the third conductive member 606 makes contact with the second and third members 602, 604 **(****FIG. 14B****)** and the measured impedance Z decreases. As the intralumenal device 608 is further advanced distally, the third conductive member 606 makes full contact with the second and third members **(****FIG. 14C****)** and the measured impedance decreases further. In that regard, the system S as shown in **FIG. 4C****,** includes an impedance subroutine 166 in a position sensing module 160. By measuring and monitoring the impedance, the system S is configured to determine the position of the proximal portion of the device 608 relative to the control handle 616 and thus the position of the distal end 608D relative to the distal end of the sheath 612 based on the configurations and geometries subroutine 156 of the memory 134 for the device 608. The stored configuration and geometry of the device 608 provides predictable and reliable determination of the location, position and/or orientation of the distal end 608D of the device 608 based on the location, position and/or orientation of the proximal portion 608P of the device 608 in relation to the control handle 616 of the guiding sheath 610 through which the device 608 extends.

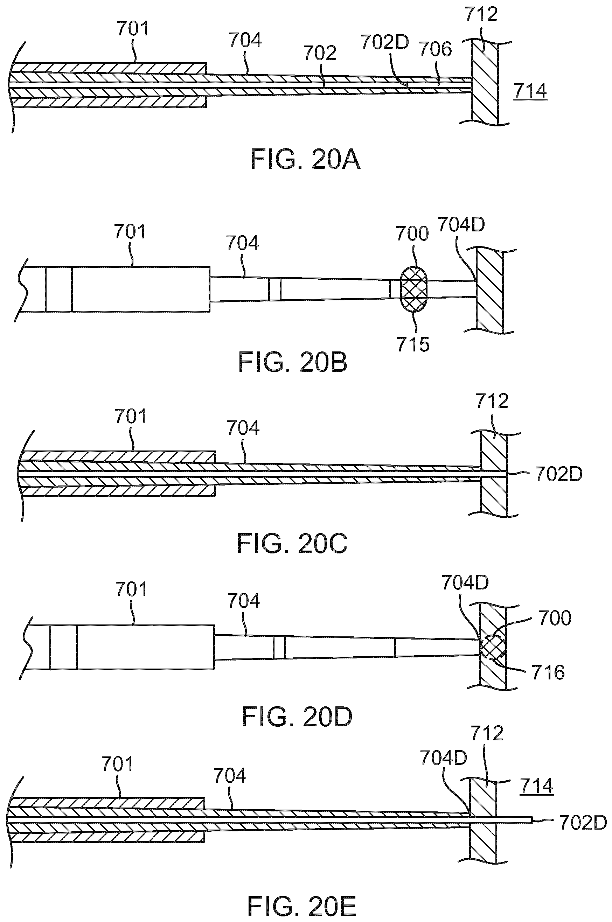

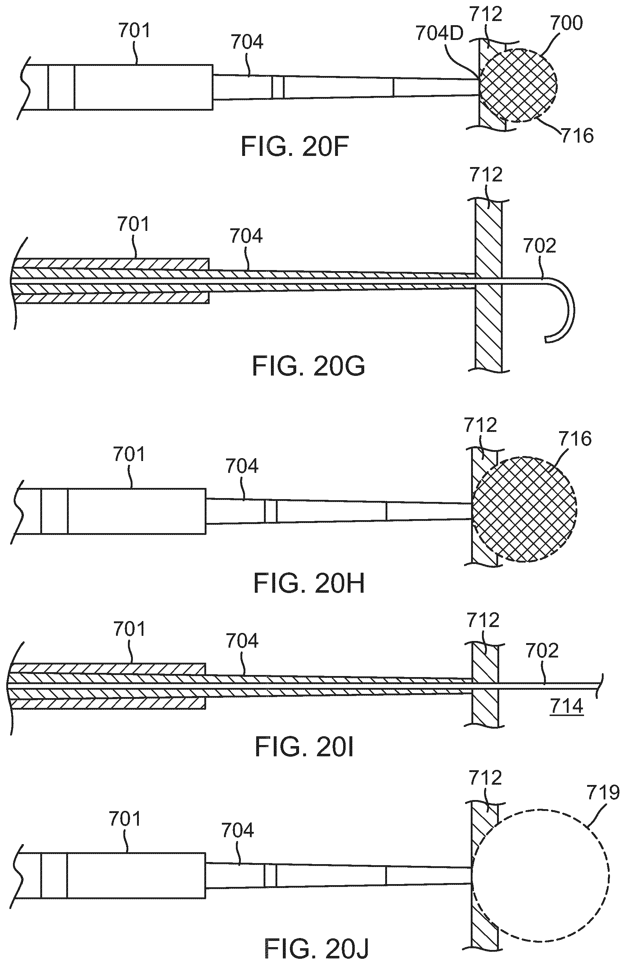

In some embodiments, the system S is configured to provide to the user on a display a visual region-of-interest indicium in real-time 3-D electroanatomical maps via an imaging electromagnetic system, such as The CARTO^{®} 3 System by Biosense Webster, Inc. Irvine, California. With reference to **FIG. 20A, FIG. 20B, FIG. 20C, FIG. 20D, FIG. 20E****,** **FIG. 20F, FIG. 20G, FIG. 20H, FIG. 20I** and **FIG. 20J****,** a generated 3-D electroanatomical visualization of an intralumenal device 702, for example, a transseptal guidewire, inserted through a lumen 706 of a lumened device 704, for example, a dilator, inserted through a guiding sheath 701, includes a visual indicium 700 that represents a region of interest ROI occupied by a protruding distal portion 702D. In some embodiments, the distal portion 702D of the intralumenal device 702 has shape memory such that it is straightened from a predetermined 2-D or 3-D configuration for insertion into and advancement through the lumen 706 and then elastically readopts the predetermined configuration when it exits the lumen or is otherwise unconfined and free from external forces. The predetermined configuration may be a "J" shape or any other 2-D or 3-D shape which is re-adopted by the distal portion 702D after the distal portion has exited the inner lumened device 704, penetrated septum 712 and distally advanced into left atrium 714.

In some embodiments, the dynamic visual indicium 700 is displayed as a graphic element near or extending from a depiction or visualization of a distal end 704D of the lumened device 704 to represent a 2D or 3D region occupied by the distal portion 702D in the left atrium. In **FIG. 20A****,** the distal portion 702D has not yet exited the lumen 706 so the distal portion is visualized on a display, as shown in **FIG. 20B****,** as a first graphic element 715, e.g., an oval, which may be displayed in a first color. As the distal portion 702D is advanced distally and pierces the septum 712, as shown in **FIG. 20C****,** the dynamic visual indicium 700 in the visualization of the distal portion 702D becomes a second graphic element 716, e.g., a circle, which may be displayed in a second color. The circle, as shown in **FIG. 20D****,** represents a spherical region that may be occupied by the distal portion 702D. Notably, a diameter of the circle is scaled to represent the length of the exposed distal portion 702D that is protruding past the distal end 704D of the lumened device 704 based on a determination of linear translation of the intralumenal device 702 by a position sensing assembly. With further distal advancement, the distal portion 702D has penetrated through the septum 712 and into the left atrium 714, as shown in **FIG. 20E****,** the circle of the second graphic element 716 increases in size as the diameter is scaled to a length representative of the greater exposed length of the distal portion 702D protruding past the distal end 704D, as shown in **FIG. 20F****.**

In some embodiments where the distal portion 704D of the intralumenal device 704 has a predetermined configuration, e.g., a "J" curvature, that is readopted by the distal portion 704D after further penetration of the septum 712 into the left atrium 714, as shown in **FIG. 20G****,** the diameter and hence the circle of the second graphic element 716 are further increased such that the circle circumscribes the exposed distal portion 702D in its entirety, as shown in **FIG. 20H****.** The circle and the spherical region represented advantageously cover all possible location of the "J" curvature regardless of its axial rotation along the longitudinal axis of the lumened device 704. In the event the distal portion 704D is further extended into the left atrium 714, e.g., beyond a predetermined threshold length past the distal end 704D, as shown in **FIG. 20I****,** the visualization of the distal portion 704D becomes a third graphic element 719, e.g., another circle of another color, whose diameter is scaled to represent the predetermined threshold length, as shown in **FIG. 20J****.** The third graphic element 719 may serve to warn the user that the distal portion 702D is "out of range." Any of the aforementioned embodiments of the position sensing assembly 40 by applying the known configurations and geometries of the intralumenal device 702, lumened device 704 and/or an outer lumened device can determine the relative linear translation of the device 702 at its proximal end to determine the relative linear translation of the device 702 at its distal end. And, the visual indicium 700 is dynamically displayed to the user in real-time with the visualization of any, some or all of these devices, so that the user has a visual cue of the region where the distal portion 702D of the intralumenal device 702 may be located at any given time during a procedure. The preceding description has been presented with reference to presently preferred embodiments of the invention. Workers skilled in the art and technology to which this invention pertains will appreciate that alterations and changes in the described structure may be practiced without meaningfully departing from the scope of the invention, which is as defined in claim 1. Any feature or structure disclosed in one embodiment may be incorporated in lieu of or in addition to other features of any other embodiments, as needed or appropriate. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims which are to have their fullest and fair scope.

## Claims

1. A system comprising:
a sheath (10) comprising a handle (16) and a catheter tube (12) extending distally from the handle;
an intralumenal device (24) comprising a shaft (28) with a predetermined geometry;
**characterised in that**:
the intralumenal device is configured to traverse through the handle and the catheter tube; and
the system further comprises a sensor assembly (40) disposed within the confines of the handle and configured to determine a parameter of the intralumenal device within the sheath to thereby determine a position of a distal end of the shaft, the parameter including a length of insertion or rotation of the intralumenal device.

2. The system of claim 1, further comprising:
a processor; and
non-transitory computer readable medium with instructions thereon, that when executed by the processor, cause the system to:
determine the length of insertion of the intralumenal device (24) within the sheath (10); and
determine the position of a distal end of the shaft based at least in part on the length of insertion of the intralumenal device within the sheath and the predetermined geometry of the shaft (28).

3. The system of claim 1, the sheath comprising a navigation sensor disposed on a distal end of the catheter tube (12).

4. The system of claim 1, a distal portion of the catheter tube (12) comprising a curvature, and a distal portion of the shaft comprising a pre-shaped curvature.

5. The system of claim 1, further comprising:
a transseptal puncture kit comprising the sheath (10) and the intralumenal device (24).

6. The system of claim 1, the intralumenal device (24) comprising a transseptal needle, or a dilator.

7. The system of claim 1, the intralumenal device (24) further comprising an identification marker (170), and the handle further comprising an identification circuit (172) configured to determine the predetermined geometry of the shaft (28) based at least in part on the identification marker, optionally the identification marker comprising a radio frequency identification (RFID) circuit.

8. The system of claim 1, the sensor assembly comprising a sensor array (46) within the handle (16) of the sheath (10) and a sensor marker disposed on a proximal portion of the shaft of the intralumenal device, the sensor array being configured to determine a position of the sensor marker within the handle to thereby determine the position of a distal end of the shaft (28).

9. The system of claim 8, the sensor marker comprising a ferromagnetic material, and the sensor array comprising a plurality of magnetic sensors.

10. The system of claim 8, the sensor array (46) comprising a plurality of sensors (44) arranged linearly along a longitudinal axis and adjacent to a lumen within the handle (16), the lumen being configured to receive the shaft (28) of the intralumenal device (24).

11. The system of claim 8, further comprising:
a processor; and
non-transitory computer readable medium with instructions thereon, that when executed by the processor, cause the system to:
determine the position of the sensor marker within the handle (16) based at least in part on an electrical signal from the sensor array (46); and
determine the position of the distal end of the shaft of the intralumenal device (24) based at least in part on the electrical signal from the sensor array and the predetermined geometry of the shaft (28).

## Patentansprüche

1. System, umfassend:
eine Hülle (10), umfassend einen Griff (16) und einen Katheterschlauch (12), der sich distal von dem Griff erstreckt;
eine intraluminale Vorrichtung (24), umfassend einen Schaft (28) mit einer vorbestimmten Geometrie;
**dadurch gekennzeichnet, dass:**
die intraluminale Vorrichtung konfiguriert ist, um durch den Griff und den Katheterschlauch hindurchzuführen; und
das System ferner eine Sensoranordnung (40) umfasst, die innerhalb der Grenzen des Griffs eingerichtet und konfiguriert ist, um einen Parameter der intraluminalen Vorrichtung innerhalb der Hülle zu bestimmen, um dadurch eine Position eines distalen Endes des Schafts zu bestimmen, wobei der Parameter eine Einführlänge oder eine Rotation der intraluminalen Vorrichtung einschließt.

2. System nach Anspruch 1, ferner umfassend:
einen Prozessor; und
nichtflüchtiges, computerlesbares Medium mit darauf befindlichen Anweisungen, die, wenn sie durch den Prozessor ausgeführt werden, das System veranlassen zum:
Bestimmen der Einführlänge der intraluminalen Vorrichtung (24) innerhalb der Hülle (10); und
Bestimmen der Position eines distalen Endes des Schafts basierend mindestens teilweise auf der Einführlänge der intraluminalen Vorrichtung innerhalb der Hülle und der vorbestimmten Geometrie des Schafts (28).

3. System nach Anspruch 1, die Hülle umfassend einen Navigationssensor, der an einem distalen Ende des Katheterschlauchs (12) eingerichtet ist.

4. System nach Anspruch 1, ein distaler Abschnitt des Katheterschlauchs (12) umfassend eine Krümmung und ein distaler Abschnitt des Schafts umfassend eine vorgeformte Krümmung.

5. System nach Anspruch 1, ferner umfassend:
ein transseptales Punktionskit, umfassend die Hülle (10) und die intraluminale Vorrichtung (24).

6. System nach Anspruch 1, die intraluminale Vorrichtung (24) umfassend eine transseptale Nadel oder einen Dilatator.

7. System nach Anspruch 1, die intraluminale Vorrichtung (24) ferner umfassend einen Identifikationsmarker (170), und der Griff ferner umfassend eine Identifikationsschaltung (172), die konfiguriert ist, um die vorbestimmte Geometrie des Schafts (28) mindestens teilweise basierend auf dem Identifikationsmarker zu bestimmen, optional der Identifikationsmarker umfassend eine Radiofrequenzidentifikationsschaltung (RFID-Schaltung).

8. System nach Anspruch 1, die Sensoranordnung umfassend ein Sensorarray (46) innerhalb des Griffs (16) der Hülle (10) und einen Sensormarker, der an einem proximalen Abschnitt des Schafts der intraluminalen Vorrichtung eingerichtet ist, wobei das Sensorarray konfiguriert ist, um eine Position des Sensormarkers innerhalb des Griffs zu bestimmen, um dadurch die Position eines distalen Endes des Schafts (28) zu bestimmen.

9. System nach Anspruch 8, der Sensormarker umfassend ein ferromagnetisches Material und das Sensorarray umfassend eine Vielzahl von Magnetsensoren.

10. System nach Anspruch 8, das Sensorarray (46) umfassend eine Vielzahl von Sensoren (44), die linear entlang einer Längsachse und angrenzend an ein Lumen innerhalb des Griffs (16) angeordnet sind, wobei das Lumen konfiguriert ist, um den Schaft (28) der intraluminalen Vorrichtung (24) aufzunehmen.

11. System nach Anspruch 8, ferner umfassend:
einen Prozessor; und
nichtflüchtiges, computerlesbares Medium mit darauf befindlichen Anweisungen, die, wenn sie durch den Prozessor ausgeführt werden, das System veranlassen zum:
Bestimmen der Position der Sensormarkierung innerhalb des Griffs (16) mindestens teilweise basierend auf einem elektrischen Signal von dem Sensorarray (46); und
Bestimmen der Position des distalen Endes des Schafts der intraluminalen Vorrichtung (24) mindestens teilweise basierend auf dem elektrischen Signal von dem Sensorarray und der vorbestimmten Geometrie des Schafts (28).

## Revendications

1. Système comprenant :
une gaine (10) comprenant une poignée (16) et un tube de cathéter (12) s'étendant distalement à partir de la poignée ;
un dispositif intraluminal (24) comprenant une tige (28) avec une géométrie prédéterminée ;
**caractérisé en ce que :**
le dispositif intraluminal est conçu pour passer à travers la poignée et le tube de cathéter ; et
le système comprend en outre un ensemble capteur (40) disposé au sein des limites de la poignée et configuré pour déterminer un paramètre du dispositif intraluminal au sein de la gaine pour déterminer de ce fait une position d'une extrémité distale de la tige, le paramètre comportant une longueur d'insertion ou une rotation du dispositif intraluminal.

2. Système selon la revendication 1, comprenant en outre :
un processeur ; et
un support non transitoire lisible par ordinateur avec des instructions sur celui-ci, qui lorsqu'elles sont exécutées par le processeur, amènent le système à :
déterminer la longueur d'insertion du dispositif intraluminal (24) au sein de la gaine (10) ; et
déterminer la position d'une extrémité distale de la tige en fonction au moins en partie de la longueur d'insertion du dispositif intraluminal au sein de la gaine et de la géométrie prédéterminée de la tige (28).

3. Système selon la revendication 1, la gaine comprenant un capteur de navigation disposé sur une extrémité distale du tube de cathéter (12).

4. Système selon la revendication 1, une portion distale du tube de cathéter (12) comprenant une courbure, et une portion distale de la tige comprenant une courbure préformée.

5. Système selon la revendication 1, comprenant en outre :
une trousse de ponction transseptale comprenant la gaine (10) et le dispositif intraluminal (24).

6. Système selon la revendication 1, le dispositif intraluminal (24) comprenant une aiguille transseptale, ou un dilatateur.

7. Système selon la revendication 1, le dispositif intraluminal (24) comprenant en outre un marqueur d'identification (170), et la poignée comprenant en outre un circuit d'identification (172) configuré pour déterminer la géométrie prédéterminée de la tige (28) en fonction au moins en partie du marqueur d'identification, facultativement le marqueur d'identification comprenant un circuit d'identification par radiofréquence (RFID).

8. Système selon la revendication 1, l'ensemble capteur comprenant un réseau de capteurs (46) au sein de la poignée (16) de la gaine (10) et un marqueur de capteur disposé sur une portion proximale de la tige du dispositif intraluminal, le réseau de capteurs étant configuré pour déterminer une position du marqueur de capteur au sein de la poignée pour déterminer de ce fait la position d'une extrémité distale de la tige (28).

9. Système selon la revendication 8, le marqueur de capteur comprenant un matériau ferromagnétique, et le réseau de capteurs comprenant une pluralité de capteurs magnétiques.

10. Système selon la revendication 8, le réseau de capteurs (46) comprenant une pluralité de capteurs (44) agencés linéairement le long d'un axe longitudinal et adjacents à une lumière au sein de la poignée (16), la lumière étant conçue pour recevoir la tige (28) du dispositif intraluminal (24).

11. Système selon la revendication 8, comprenant en outre :
un processeur ; et
un support non transitoire lisible par ordinateur avec des instructions sur celui-ci, qui lorsqu'elles sont exécutées par le processeur, amènent le système à :
déterminer la position du marqueur de capteur au sein de la poignée (16) en fonction au moins en partie d'un signal électrique provenant du réseau de capteurs (46) ; et
déterminer la position de l'extrémité distale de la tige du dispositif intraluminal (24) en fonction au moins en partie du signal électrique provenant du réseau de capteurs et de la géométrie prédéterminée de la tige (28).
